(19) Europäisches Patentamt
European Patent Office
Office européen des brevets

(11) Veröffentlichungsnummer: **0 282 898**
**A2**

(12) **EUROPÄISCHE PATENTANMELDUNG**

(21) Anmeldenummer: **88103738.6**

(22) Anmeldetag: **09.03.88**

(51) Int. Cl.⁴: **C07C 49/84**, C07C 59/90, C07C 69/738, C07C 103/76, C07C 69/734, C07C 59/66, C07C 59/64, C07C 49/835, C07D 317/60

Patentansprüche für folgende Vertragsstaaten: ES + GR.

(30) Priorität: **20.03.87 CH 1072/87**

(43) Veröffentlichungstag der Anmeldung: **21.09.88 Patentblatt 88/38**

(84) Benannte Vertragsstaaten: **AT BE CH DE ES FR GB GR IT LI LU NL SE**

(71) Anmelder: **F. HOFFMANN-LA ROCHE & CO. Aktiengesellschaft**

**CH-4002 Basel(CH)**

(72) Erfinder: **Fischli, Albert, Prof. Dr.**
**Im Wenkenberg 20**
**CH-4125 Riehen(CH)**
Erfinder: **Gutknecht, Eva-Maria, Dr.**
**Im Feldele 23**
**D-7845 Buggingen-Seefelden(DE)**
Erfinder: **Obrecht, Daniel, Dr.**
**Frohburgstrasse 45**
**CH-4052 Basel(CH)**

(74) Vertreter: **Lederer, Franz, Dr. et al**
**Patentanwält Dr. Franz Lederer Lucile Grahnstrasse 22**
**D-8000 München 80(DE)**

(54) **Propiolophenonderivate.**

(57) Propiolophenonderivative der Formel

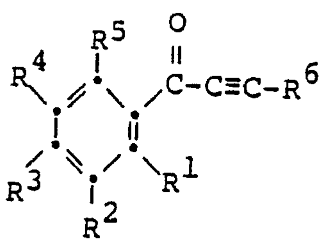

I

worin
$R^6$ Wasserstoff, niederes Alkyl oder einen Rest der Formel

EP 0 282 898 A2

$-COOR^7$, (a)

$-CONR^8R^9$, (b)

$-C(R^{10})=O$, (c)

$-C(R^{11})(OR^{12})_2$, (d)

$-C(OR^{13})_3$ (e) oder

$-C(R^{14})(R^{15})OR^{16}$ (f)

bedeutet;
sowie entsprechende Hydroxyverbindungen der Formel

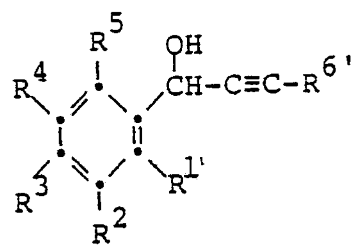

II

worin und $R^{6'}$ Wasserstoff, niederes Alkyl, einen Rest der Formel (a), (b), (c), (d) oder (e) oder einen Rest der Formel

$-C(R^{14})(R^{15})OR^{16'}$ (f')

bedeutet;
wobei die übrigen Symbole die in Beschreibung und Ansprüchen angegebene Bedeutung besitzen; zeigen mucosaprotektive und/oder magensäuresekretionshemmende Eigenschaften, so dass sie zur Bekämpfung oder Verhütung von Krankheiten des Magen-Darm-Trakts verwendet werden können, insbesondere gegen Ulcus ventriculi und/oder duodeni.

# Propiolophenonderivate

Die vorliegende Erfindung betrifft Propiolophenonderivate. Im speziellen betrifft sie Propiolophenonderivate der allgemeinen Formel

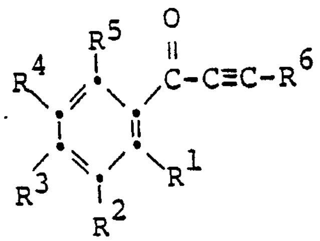

I

worin

$R^1$, $R^2$, $R^3$, $R^4$ und $R^5$ je Wasserstoff, Halogen, niederes Alkyl, niederes Alkoxy-niederes-alkyl, Hydroxy, niederes Alkoxy, niederes Alkenyloxy, niederes Alkinyloxy, niederes Alkoxy-niederes-alkoxy, Acyloxy, Aryl-niederes-alkoxy, niederes Alkylthio, niederes Alkoxy-niederes-alkylthio, niederes Alkenylthio, niederes Alkinylthio, Aryl-niederes-alkylthio, gegebenenfalls substituiertes Amino oder Trifluormethyl oder zwei benachbarte dieser Substituenten gemeinsam und zusammen mit den Kohlenstoffatomen, an welche sie gebunden sind, einen 5-bis 7-gliedrigen Ring bedeuten, wobei von den Substituenten $R^1$ bis $R^5$ mindestens zwei Wasserstoff bedeuten und mindestens einer von Wasserstoff verschieden ist; und

$R^6$ Wasserstoff, niederes Alkyl oder einen Rest der Formel

$-COOR^7$, (a) $-CONR^8R^9$, (b) $-C(R^{10})=O$, (c)

$-C(R^{11})(OR^{12})_2$, (d) $-C(OR^{13})_3$ (e) oder

$-C(R^{14})(R^{15})OR^{16}$ (f) bedeutet;

$R^7$ Wasserstoff, niederes Alkyl, niederes Alkenyl, niederes Alkoxy-niederes-alkyl, niederes Alkoxy-niederes-alkoxy-niederes-alkyl, Aryl oder Aryl-niederes-alkyl bedeutet;

$R^8$ und $R^9$ je Wasserstoff oder niederes Alkyl oder gemeinsam und zusammen mit dem Stickstoffatom einen 5-bis 7-gliedrigen gesättigten heterocyclischen Rest bedeuten;

$R^{10}$ Wasserstoff, niederes Alkyl, Aryl oder Aryl-niederes-alkyl bedeutet;

$R^{11}$ Wasserstoff, niederes Alkyl, Aryl oder Aryl-niederes-alkyl bedeutet;

$R^{12}$ niederes Alkyl oder niederes Alkoxy-niederes-alkyl bedeutet;

$R^{13}$ niederes Alkyl bedeutet;

$R^{14}$ und $R^{15}$ je Wasserstoff, niederes Alkyl, Aryl oder Aryl-niederes-alkyl bedeuten; und

$R^{16}$ Wasserstoff, niederes Alkyl, niederes Alkoxy-niederes-alkyl, niederes Alkenyl, niederes Alkinyl, Acyl oder Aryl-niederes-alkyl bedeuten;

sowie pharmazeutisch verwendbare Salze von sauren Verbindungen der Formel I mit Basen und von basischen Verbindungen der Formel I mit Säuren.

Diese Verbindungen sind neu, mit Ausnahme von

- 4-Methoxy-1-(3-methylphenyl)-2-butin-1-on;

- 4-Methoxy-1-(4-methoxyphenyl)-4-methyl-2-pentin-1-on;
- 4-Methoxy-4-methyl-1-(3-methylphenyl)-2-pentin-1-on;
- 3-(2,6-Dimethoxybenzoyl)propiolsäuremethylester und
- N,N-Diisopropyl-3-(2-hydroxy-5-methylbenzoyl)propiolamid,

und es hat sich gezeigt, dass sie wertvolle pharmakodynamische Eigenschaften besitzen, nämlich mucosaprotektive und/oder magensäuresekretionshemmende Eigenschaften, so dass sie zur Bekämpfung oder Verhütung von Krankheiten des Magen-Darm-Trakts verwendet werden können, insbesondere gegen Ulcus ventriculi und/oder duodeni.

Gegenstand der vorliegenden Erfindung sind in erster Linie die eingangs definierten Verbindungen und Salze als therapeutische Wirkstoffe, Arzneimittel, enthaltend eine solche Verbindung oder ein Salz davon, die Herstellung solcher Arzneimittel, die Verwendung der eingangs definierten Verbindungen und Salze bei der Bekämpfung bzw. Verhütung von Krankheiten, insbesondere bei der Bekämpfung bzw. Verhütung von Ulcus ventriculi und/oder duodeni bzw. die Verwendung der eingangs definierten Verbindungen und Salze zur Herstellung von Arzneimitteln gegen Ulcus ventriculi und/oder duodeni, weiterhin die neuen unter den eingangs definierten Verbindungen und Salzen als solche sowie Verfahren für die Herstellung dieser neuen Verbindungen und Salze.

Der Ausdruck "nieder" bezeichnet Verbindungen oder Reste mit höchstens 7, vorzugsweise höchstens 4 Kohlenstoffatomen.

Der Ausdruck "Alkyl" bezeichnet geradkettige oder verzweigte gesättigte Kohlenwasserstoffreste, wie Methyl, Aethyl, n-Butyl und dergleichen. Die Ausdrücke "Alkoxy" und "Alkylthio" bezeichnen über ein Sauerstoffatom bzw. ein Schwefelatom gebundene Alkylgruppen im Sinne der vorstehenden Definition, wie Methoxy bzw. Methylthio und dergleichen. Die Ausdrücke "Alkenyl" und "Alkinyl" bezeichnen Kohlenwasserstoffreste, welche eine Kohlenstoff-Kohlenstoff-Doppel-oder -Dreifachbindung enthalten, z.B. also Reste wie Dimethylallyl. Der Ausdruck "Aryl" bezeichnet einen gegebenenfalls substituierten Phenylrest, wie 4-Methoxyphenyl, 3,4-Methylendioxyphenyl, 2,4,6-Trimethylphenyl, 3,4,5-Trimethoxyphenyl und dergleichen. Der Ausdruck "Acyl" umfasst niedere Alkanoylgruppen, wie Acetyl oder dergleichen, und Aroylgruppen (d.h. Arylcarbonylgruppen), wie 3,4,5-Trimethoxybenzoyl und dergleichen. Der Ausdruck "Halogen" umfasst die vier Formen Chlor, Fluor, Brom und Jod. Der Ausdruck "gegebenenfalls substituiertes Amino" bezeichnet eine Aminogruppe, die durch niederes Alkyl oder Acyl monosubstituiert oder durch niederes Alkyl und Acyl oder durch zwei niedere Alkylreste disubstituiert sein kann.

Der 5-bis 7-gliedrige Ring, den zwei benachbarte der Substituenten $R^1$, $R^2$, $R^3$, $R^4$, $R^5$ zusammen mit den Kohlenstoffatomen, an welche sie gebunden sind, bilden können, kann heterocyclisch oder carbocyclisch sein, er kann gegebenenfalls eine oder mehrere zusätzliche Doppelbindungen enthalten, wobei er aromatisch oder nicht-aromatisch sein kann, und er kann substituiert oder unsubstituiert sein. Der 5-bis 7-gliedrige gesättigte heterocyclische Rest, den $R^8$ und $R^9$ zusammen mit dem Stickstoffatom bilden können, kann ein zusätzliches Heteroatom enthalten, und er kann substituiert oder unsubstituiert sein.

Beispielsweise können $R^1$, $R^2$, $R^3$, $R^4$ und $R^5$ je Wasserstoff, Halogen, niederes Alkyl, Hydroxy, niederes Alkoxy, niederes Alkylthio oder di-niederes Alkylamino oder zwei benachbarte dieser Substituenten zusammen niederes Alkylen oder niederes Alkylendioxy bedeuten, wobei von den Substituenten $R^1$ bis $R^5$ mindestens einer von Wasserstoff verschieden ist und mindestens zwei Wasserstoff bedeuten; dabei bedeutet mit Vorteil $R^1$ Wasserstoff.

In Formel I bedeuten zweckmässigerweise $R^1$ und $R^2$ je Wasserstoff und $R^3$, $R^4$ und $R^5$ je niederes Alkoxy oder $R^1$ und $R^5$ je Wasserstoff und $R^2$, $R^3$ und $R^4$ je niederes Alkoxy oder $R^1$, $R^3$ und $R^4$ je Wasserstoff und $R^2$ und $R^5$ je niederes Alkoxy oder $R^1$, $R^2$ und $R^5$ je Wasserstoff und $R^3$ und $R^4$ zusammen niederes Alkylendioxy oder niederes Alkylen oder $R^1$, $R^2$, $R^4$ und $R^5$ je Wasserstoff und $R^3$ Halogen, niederes Alkyl, Hydroxy, niederes Alkoxy, niederes Alkylthio oder di-niederes Alkylamino; $R^6$ bedeutet zweckmässigerweise einen Rest der Formel (a), (b), (c), (d), (e) oder (f), wobei $R^7$ Wasserstoff, niederes Alkyl, niederes Alkoxy-niederes-alkoxy-niederes-alkyl, Aryl-niederes Alkyl oder niederes Alkenyl, $R^8$ und $R^9$ je niederes Alkyl, $R^{10}$ Wasserstoff, $R^{11}$ Wasserstoff, $R^{12}$ niederes Alkyl, $R^{13}$ niederes Alkyl, $R^{14}$ Wasserstoff, $R^{15}$ Wasserstoff oder niederes Alkyl und $R^{16}$ Wasserstoff, niederes Alkenyl, niederes Alkoxy-niederes-alkyl oder Acyl bedeuten.

Unter den Verbindungen der Formel I sind diejenigen bevorzugt, worin $R^1$ und $R^2$ je Wasserstoff und $R^3$, $R^4$ und $R^5$ je Methoxy oder $R^1$ und $R^5$ je Wasserstoff und $R^2$, $R^3$ und $R^4$ je Methoxy oder $R^1$, $R^3$ und $R^4$ je Wasserstoff und $R^2$ und $R^5$ je Methoxy oder $R^1$, $R^2$ und $R^5$ je Wasserstoff und $R^3$ und $R^4$ zusammen Methylendioxy oder $R^1$, $R^2$, $R^4$ und $R^5$ je Wasserstoff und $R^3$ Chlor, Fluor, Methyl, Hydroxy, Methoxy oder Methylthio bedeuten und worin $R^6$ einen Rest der Formel (a), (b) oder (f) bedeutet, wobei $R^7$ Wasserstoff, Methyl oder Methoxyäthoxyäthyl, $R^8$ und $R^9$ je Methyl, $R^{14}$ Wasserstoff, $R^{15}$ Wasserstoff oder Methyl und $R^{16}$ Wasserstoff oder 1-Aethoxyäthyl bedeuten.

Eine ganz besonders bevorzugte Verbindung der Formel I ist die 3-[3,4-(Methylendioxy)benzoyl]-propiolsäure. Ebenfalls besonders bevorzugte Verbindungen der Formel I sind:
4-Hydroxy-1-(3,4,5-trimethoxyphenyl)-2-butin-1-on;
3-(4-Methoxybenzoyl)propiolsäure;
3-(2,3,4-Trimethoxybenzoyl)propiolsäuremethylester;
3-(4-Hydroxybenzoyl)propiolsäuremethylester;
3-(3,4,5-Trimethoxybenzoyl)propiolsäure-[2-(2-methoxyäthoxy)äthylester;
3-(4-Methoxybenzoyl)propiolsäure-[2-(2-methoxyäthoxy)äthyl]ester;
3-(2,5-Dimethoxybenzoyl)propiolsäuremethylester;
3-(2,5-Dimethoxybenzoyl)propiolsäure; und
3-[3,4-(Methylendioxy)benzoyl]propiolsäure-[2-(2-methoxyäthoxy)äthyl]ester.

Weitere bevorzugte Verbindungen der Formel I sind:
3-(2,3,4-Trimethoxybenzoyl)propiolsäure;
3-(3,4,5-Trimethoxybenzoyl)propiolsäure;
3-(4-Fluorbenzoyl)propiolsäuremethylester;
3-(3,4,5-Trimethoxybenzoyl)propiolsäuremethylester;
N,N-Dimethyl-3-(3,4,5-trimethoxybenzoyl)propiolamid;
1-(4-Fluorphenyl)-4-hydroxy-2-butin-1-on;
4-Hydroxy-1-(4-methylphenyl)-2-butin-1-on;
4-(1-Aethoxyäthoxy)-1-(4-fluorphenyl)-2-butin-1-on;
1-(4-Chlorphenyl)-4-hydroxy-2-butin-1-on;
4-Hydroxy-1-(4-methoxyphenyl)-2-butin-1-on;
4-Hydroxy-1-[3,4-(methylendioxy)phenyl]-2-butin-1-on;
4-Hydroxy-1-(3,4,5-trimethoxyphenyl)-2-pentin-1-on;
4-(1-Aethoxyäthoxy)-1-(2,3,4-trimethoxyphenyl)-2-butin-1-on;
4-Hydroxy-1-(2,3,4-trimethoxyphenyl)-2-butin-1-on; und
4-Hydroxy-1-[4-(methylthio)phenyl]-2-butin-1-on.

Die neuen Propiolophenonderivate der eingangs definierten Formel I und deren Salze können erfindungsgemäss dadurch hergestellt werden, dass man

a) eine Verbindung der allgemeinen Formel

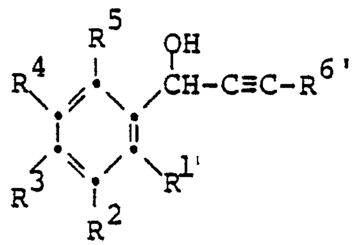

II

worin $R^1$, $R^2$, $R^3$, $R^4$ und $R^5$ obige Bedeutung besitzen und $R^{6'}$ Wasserstoff, niederes Alkyl, einen Rest der Formel (a), (b), (c), (d) oder (e) oder einen Rest der Formel

$$-C(R^{14})(R^{15})OR^{16'} \quad (f')$$

bedeutet, worin $R^{14}$ und $R^{15}$ obige Bedeutung besitzen und $R^{16'}$ die oben für $R^{16}$ definierte Bedeutung besitzt, jedoch nicht Wasserstoff bedeutet wenn $R^{14}$ und/oder $R^{15}$ Wasserstoff bedeutet, oxydiert; oder

b) von einer Verbindung der allgemeinen Formel

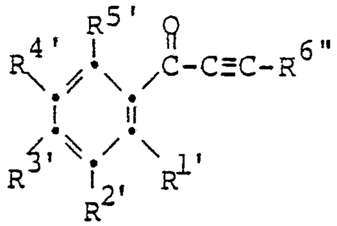

III

worin $R^{1'}$, $R^{2'}$, $R^{3'}$, $R^{4'}$ und $R^{5'}$ die oben für $R^1$, $R^2$, $R^3$, $R^4$ und $R^5$ angegebene Bedeutung besitzen und maximal drei davon zusätzlich geschütztes Hydroxy, geschütztes Amino oder geschütztes niederes Alkylamino bedeuten können, $R^{6''}$ einen Rest der Formel (a), (b), (c), (d), (e) oder

$-C(R^{14})(R^{15})OR^{16''}$ (f'')

bedeutet, worin $R^{14}$ und $R^{15}$ obige Bedeutung besitzen und $R^{16''}$ die oben für $R^{16}$ angegebene Bedeutung besitzt und zusätzlich eine Schutzgruppe bedeuten kann, wobei das Molekül mindestens eine Schutzgruppe enthält,
die Schutzgruppe(n) abspaltet; oder

c) eine Verbindung der allgemeinen Formel

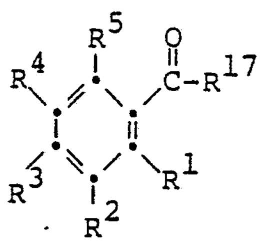

IV

worin $R^1$, $R^2$, $R^3$, $R^4$ und $R^5$ obige Bedeutung besitzen und $R^{17}$ eine Abgangsgruppe bedeutet, mit einer Verbindung der allgemeinen Formel

$HC{\equiv}C-R^{6'''}$ V

worin $R^{6'''}$ einen Rest der Formel (a), (b), (d), (e) oder (f) bedeutet,
umsetzt; oder

d) eine Verbindung der Formel I, worin $R^6$ einen Rest der Formel (a) bedeutet, wobei $R^7$ von Wasserstoff verschieden ist, zur entsprechenden Carbonsäure spaltet; oder

e) eine Verbindung der Formel I, worin $R^6$ einen Rest der Formel (f) bedeutet, wobei $R^{16}$ Wasserstoff bedeutet, acyliert; oder

f) eine Verbindung der Formel I, worin $R^6$ einen Rest der Formel (d) bedeutet, in die entsprechende Verbindung der Formel I, worin $R^6$ einen Rest der Formel (c) bedeutet, überführt; oder

g) eine saure Verbindung der Formel I mit einer Base bzw. eine basische Verbindung der Formel I mit einer Säure in ein pharmazeutisch verwendbares Salz überführt.

Die Verbindungen der allgemeinen Formel II haben ähnliche pharmakodynamische Eigenschaften wie die Propiolophenonderivate der allgemeinen Formel I, und zwar in erster Linie diejenigen, worin $R^{6'}$ einen Rest der obigen Formel (a) bedeutet, wobei $R^7$ insbesondere niederes Alkyl oder niederes Alkoxy-niederes-alkoxy-niederes-alkyl bedeutet; repräsentative Beispiele derartiger Verbindungen sind der 4-Hydroxy-4-[3,4-methylendioxy)phenyl] -2-butinsäuremethylester und der 4-Hydroxy-4-(3,4,5-trimethoxyphenyl)-2-butinsäure-[2-(2-methoxyäthoxy)äthyl]ester. Die Verbindungen der Formel II sind neu, mit Ausnahme von
- 4-(3,5-Dimethoxyphenyl)-4-hydroxy-2-butinsäureäthylester;
- 4-(2,5-Dimethoxyphenyl)-4-hydroxy-2-butinsäureäthylester;
- 4-(2-Methoxyphenyl)-4-hydroxy-2-butinsäureäthylester;
- 4-(2-Benzyloxy-6-methoxyphenyl)-4-hydroxy-2-butinsäuremethylester;
- 4-(2-Benzyloxy-6-methoxyphenyl)-4-hydroxy-2-butinsäure; und
- 4-(2,6-Dimethoxyphenyl)-4-hydroxy-2-butinsäure.

Gegenstand der vorliegenden Erfindung sind demnach auch Verbindungen der Formel II als therapeutische Wirkstoffe, Arzneimittel, enthaltend eine solche Verbindung, die Herstellung solcher Arzneimittel, die Verwendung der Verbin dungen der Formel II bei der Bekämpfung bzw. Verhütung von Krankheiten, insbesondere bei der Bekämpfung bzw. Verhütung von Ulcus ventriculi und/oder duodeni bzw. die Verwendung der Verbindungen der Formel II zur Herstellung von Arzneimitteln gegen Ulcus ventriculi und/oder duodeni, weiterhin die neuen unter den Verbindungen der Formel II als solche und die Herstellung dieser neuen Verbindungen.

In Formel II bedeuten zweckmässigerweise $R^1$ und $R^2$ je Wasserstoff und $R^3$, $R^4$ und $R^5$ je niederes Alkoxy oder $R^1$ und $R^5$ je Wasserstoff und $R^2$, $R^3$ und $R^4$ je niederes Alkoxy oder $R^1$, $R^2$ und $R^5$ je Wasserstoff und $R^3$ und $R^4$ zusammen niederes Alkylendioxy oder niederes Alkylen oder $R^1$, $R^2$, $R^4$ und $R^5$ je Wasserstoff und $R^3$ Halogen, niederes Alkyl, Hydroxy, niederes Alkoxy, niederes Alkylthio oder di-niederes Alkylamino.

Die neuen Verbindungen der Formel II können erfindungsgemäss dadurch hergestellt werden, dass man

aa) eine Verbindung der allgemeinen Formel

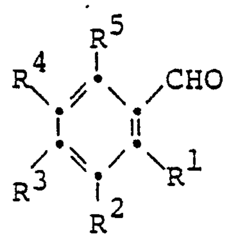

VI

worin $R^1$, $R^2$, $R^3$, $R^4$ und $R^5$ obige Bedeutung besitzen,
mit einer Verbindung der Formel

$HC{\equiv}C\text{-}R^{6iv}$ Va

worin $R^{6iv}$ einen Rest der obigen Formel (a), (b), (d), (e) oder (f') bedeutet,
umsetzt; oder

bb) von einer Verbindung der allgemeinen Formel

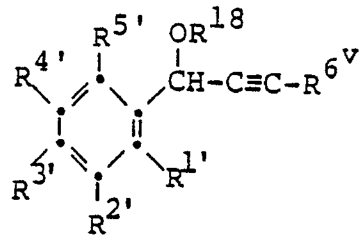

VII

worin $R^{1'}$, $R^{2'}$, $R^{3'}$, $R^{4'}$ und $R^{5'}$ obige Bedeutung besitzen, $R^{6v}$ einen Rest der obigen Formel (a), (b) oder (c) oder einen Rest der Formel

$-C(R^{14})(R^{15})OR^{16'''}$ (f''')

bedeutet, wobei $R^{7}$ und $R^{10}$ von Wasserstoff verschieden sind, $R^{14}$ und $R^{15}$ die in Formel II und $R^{16'''}$ die in Formel II für $R^{16'}$ angegebene Bedeutung besitzen, wobei aber $R^{16'''}$ zusätzlich noch eine Schutzgruppe bedeuten kann, und $R^{18}$ eine Schutzgruppe bedeutet,
die Schutzgruppe(n) abspaltet.

Die Oxidation gemäss Verfahrensvariante a) erfolgt nach an sich bekannten Methoden, welche jedem Fachmann, der sich die Aufgabe stellt, eine Hydroxygruppe in eine Oxogruppe überzuführen, geläufig sind. Als Oxidationsmittel verwendet man zweckmässigerweise Mangandioxid (Braunstein) in einem hierfür geeigneten, unter den Reaktionsbedingungen inerten Lösungsmittel, beispielsweise in einem halogenierten Kohlenwasserstoff, wie Methylenchlorid oder dergleichen. Die Oxidation mittels Mangandioxid erfolgt

zweckmässigerweise in einem Temperaturbereich von etwa 0° bis etwa Raumtemperatur und dauert, je nach den übrigen Bedingungen, ca. 10 Minuten bis ca. 20 Stunden. Bedeutet $R^{6'}$ in Formel II einen Rest der Formel (c), worin $R^{10}$ Wasserstoff bedeutet, dann kann je nach den Reaktionsbedingungen Oxidation zu einem Rest der Formel (a), worin $R^7$ Wasserstoff bedeutet, erfolgen.

Als Schutzgruppen in den Verbindungen der allgemeinen Formel III, welche als Ausgangsprodukte in Verfahrensvariante b) verwendet werden, eignen sich selbstverständlich nur solche, welche durch Methoden abgespalten werden können, bei welchen diese Schutzgruppen selektiv entfernt werden, ohne dass andere im Molekül vorhandene Strukturelemente in Mitleidenschaft gezogen werden. Die Entfernung der Schutzgruppe bzw. der Schutzgruppen aus den Verbindungen der allgemeinen Formel III erfolgt nach an sich bekannten Methoden, wobei natürlich für die Wahl der zur Anwendung gelangenden Methode die Natur der zu entfernenden Schutzgruppe bzw. Schutzgruppen in Betracht gezogen werden muss und zu beachten ist, dass nur die Schutzgruppe bzw. Schutzgruppen selektiv entfernt, andere im Molekül vorhandene Strukturelemente jedoch nicht in Mitleidenschaft gezogen werden sollen. Als O-Schutzgruppen eignen sich beispielsweise leicht abspaltbare Acetal-und Ketalschutzgruppen, wie Methoxymethyl, Methoxyäthoxymethyl, 1-Aethoxyäthyl, 2-(Trimethylsilyl)äthoxymethyl, Tetrahydro-2H-pyran-2-yl und dergleichen; leicht abspaltbare metallorganische Gruppen, insbesondere Trialkylsilylgruppen, wie Trimethylsilyl, t-Butyldimethylsilyl und dergleichen; leicht abspaltbare Aralkylgruppen, wie Triphenylmethyl und dergleichen; leicht abspaltbare Acylgruppen, wie Acetyl und dergleichen; usw. Als N-Schutzgruppen eignen sich in erster Linie leicht abspaltbare Acylgruppen, wie t-Butyloxycarbonyl und dergleichen.

Methoden für die Entfernung der Reste, welche vorstehend als Beispiele für Schutzgruppen erwähnt wurden, sind in der Literatur beschrieben und demnach jedem Fachmann geläufig. So kann man beispielsweise die Methoxymethylgruppe, die Methoxyäthoxymethylgruppe, die 1-Aethoxyäthylgruppe, die 2-(Trimethylsilyl)äthoxymethylgruppe, die Tetrahydro-2H-pyran-2-ylgruppe, die Trimethylsilylgruppe, die t-Butyldi methylsilylgruppe und die Triphenylmethylgruppe unter sauren Bedingungen abspalten, beispielsweise mittels wässriger Salzsäure in einem unter den Reaktionsbedingungen inerten organischen Lösungsmittel, wie Tetrahydrofuran, die Tetrahydro-2H-pyran-2-ylgruppe, die Trimethylsilylgruppe und die t-Butyldimethylsilylgruppe aber zweckmässigerweise auch mittels Pyridinium-p-toluolsulfonat in einem unter den Reaktionsbedingungen inerten organischen Lösungsmittel oder Lösungsmittelgemisch, wie Tetrahydrofuran/Aethanol, und die Trimethylsilylgruppe und die t-Butyldimethylsilylgruppe auch mittels eines quartären Ammoniumfluorids, wie Tetrabutylammoniumfluorid, in einem unter den Reaktionsbedingungen inerten organischen Lösungsmittel, wie Tetrahydrofuran. Die Acetylgruppe kann unter milden alkalischen Bedingungen abgespalten werden, beispielsweise mittels verdünnter (ca. 2-5%iger) Kalilauge in einem unter den Reaktionsbedingungen inerten organischen Lösungsmittel, wie Tetrahydrofuran. Die Abspaltung einer t-Butyloxycarbonylgruppe kann unter sauren Bedingungen erfolgen, z.B. mittels einer wässrigen Säure oder mittels wasserfreier Trifluoressigsäure.

Als Abgangsgruppe ($R^{17}$) in den Verbindungen der Formel IV, welche in Verfahrensvariante (c) als Ausgangsprodukte verwendet werden, eignen sich in erster Linie Reste wie N-Methoxy-N-methylamino und dergleichen. Die Umsetzung der Verbindungen der Formeln IV und V erfolgt in Gegenwart einer starken Base, wie Butyllithium, Alkylmagnesiumhalogenide (z.B. Aethylmagnesiumbromid) und dergleichen, in einem unter den Reaktionsbedingungen inerten organischen Lösungsmittel oder Lösungsmittelgemisch, beispielsweise in Tetrahydrofuran/Hexamethylphosphortriamid. Weiterhin erfolgt die Umsetzung zweckmässigerweise bei einer Temperatur von etwa -80° bis etwa Raumtemperatur und dauert ca. 1 Stunde bis ca. 3 Stunden.

Die Esterspaltung gemäss Verfahrensvariante (d) erfolgt nach allgemein bekannten und jedem Fachmann geläufigen Methoden, zweckmässigerweise durch Hydrolyse mittels einer starken anorganischen Base, beispielsweise eines Alkalimetallhydroxids, wie Kaliumhydroxid oder dergleichen, in einem geeigneten Lösungsmittelsystem, beispielsweise in Wasser oder wässrigem Tetrahydrofuran und dergleichen. Falls $R^7$ einen unter sauren Bedingungen leicht abspaltbaren Benzylrest wie 4-Methoxybenzyl, 3,4-Methylendioxybenzyl, 2,4,6-Trimethylbenzyl oder dergleichen bedeutet, so kann die Esterspaltung zweckmässigerweise auch mittels Trifluoressigsäure (in An-oder Abwesenheit eines Lösungsmittels, wie Methylenchlorid, Anisol oder dergleichen), mittels Ameisensäure, mittels Bromwasserstoff in Eisessig oder mittels analoger Reagenzien erfolgen.

Auch die Acylierung gemäss Verfahrensvariante (e) erfolgt nach allgemein üblichen Methoden. Als Acylierungsmittel verwendet man beispielsweise ein dem einzuführenden Acylrest entsprechendes Säurehalogenid, wie Acetylchlorid, 3,4,5-Trimethoxybenzoylchlorid und dergleichen. Die Acylierung mittels eines derartigen Säurehalogenids erfolgt zweckmässigerweise in Gegenwart einer Base, insbesondere einer tertiären organischen Base, wie Pyridin, Triäthylamin, N-Methylpiperidin, 4-Dimethylaminopyridin oder dergleichen. Als Lösungsmittel eignen sich in erster Linie halogenierte Kohlenwasserstoffe, wie Methylenchlorid oder dergleichen; falls als Base Pyridin verwendet wird, so kann dieses gleichzeitig auch als Lösungsmittels dienen. Bedeuten eines oder mehrere von $R^1$-$R^5$ eine Hydroxygruppe und/oder eine Aminogruppe und/oder eine niedere Alkylaminogruppe, so wird bzw. werden diese ebenfalls acyliert.

Gemäss Verfahrensvariante (f) wird eine Acetal-oder Ketalgruppe in eine Carbonylgruppe übergeführt. Dies erfolgt nach allgemein üblichen und jedem Fachmann geläufigen Methoden, zweckmässigerweise mittels wässriger Perchlorsäure oder dergleichen, in einem unter den Reaktionsbedingungen inerten organischen Lösungsmittel, wie Dioxan oder der gleichen, bei etwa Raumtemperatur und dauert einige (beispielsweise 2) Stunden.

Die Ueberführung einer sauren Verbindung der Formel I in ein pharmazeutisch verwendbares Salz kann durch Behandeln mit einer geeigneten Base in an sich bekannter Weise bewerkstelligt werden. Als derartige Salze eignen sich sowohl solche mit von einer anorganischen Base abgeleiten Kationen, z.B. also Kaliumsalze, Natriumsalze, Calciumsalze und dergleichen, als auch Salze mit organischen Basen, wie Aethylendiamin, Monoäthanolamin, Diäthanolamin und dergleichen.

Die Ueberführung einer basischen Verbindung der Formel I in ein pharmazeutisch verwendbares Salz kann durch Behandeln mit einer geeigneten Säure bewerkstelligt werden. Als derartige Salze eignen sich sowohl solche mit anorganischen Säuren, wie Chlorwasserstoff, Bromwasserstoff, Phosphorsäure, Schwefelsäure und dergleichen, als auch Salze mit organischen Säuren, wie Zitronensäure, Aepfelsäure, Methansulfonsäure, p-Toluolsulfonsäure und dergleichen.

Die Herstellung von Verbindungen der Formel II gemäss Verfahrensvariante aa) erfolgt in Gegenwart einer starken Base, wie Butyllithium, Alkylmagnesiumhalogenide (z.B. Aethylmagnesiumbromid) und dergleichen, in einem unter den Reaktionsbedingungen inerten organischen Lösungsmittel oder Lösungsmittelgemisch, beispielsweise in Tetrahydrofuran/n-Hexan, Tetrahydrofuran/Diäthyläther/n-Hexan und dergleichen. Die Reaktionstemperatur liegt zweckmässigerweise in einem Bereich von etwa -120° bis etwa Raumtemperatur, wobei die Wahl der Reaktionstemperatur unter anderem auch von dem verwendeten Lösungsmittel oder Lösungsmittelgemisch abhängt; die Reaktionsdauer beträgt etwa 1-2 Stunden. Die Verbindungen der Formel Va können in freier Form oder in Form reaktionsfähiger Derivate verwendet werden, zweckmässigerweise in Form von Tri-nieder-alkylsilylderivaten, z.B. in Form von Trimethylsilylderivaten. Beispiele geeigneter Verbindungen der Formel Va bzw. reaktionsfähiger Derivate davon sind 1-(1-Aethoxyäthoxy)-2-propin, Propiolsäuremethylester, Propiolsäure, 3,3-Diäthoxy-1-propin, 3-Butin-2-ol, Propiolsäureäthylester, 1-[(3-Methyl-2-butenyl)oxy]-2-propin, 3-Trimethylsilyl-orthopropiolsäure-triäthylester u.s.w.

Als Schutzgruppen ($R^{18}$) in den Verbindungen der Formel VII, welche gemäss Verfahrensvariante bb) als Ausgangsprodukte verwendet werden, eignen sich in erster Linie leicht abspaltbare metallorganische Gruppen, wie Trimethylsilyl, t-Butyldimethylsilyl und dergleichen. Derartige Reste werden zweckmässigerweise mittels wässriger Säure abgespalten, beispielsweise mittels wässriger Salzsäure in einem unter den Reaktionsbedingungen inerten organischen Lösungsmittel, wie Essigester, Dioxan oder dergleichen; die Abspaltung solcher Gruppen kann aber zweckmässigerweise auch mittels Pyridinium-p-toluolsulfonat oder mittels eines quartären Ammoniumfluorids, wie Tetrabutylammoniumfluorid erfolgen.

Die Herstellung der Ausgangsprodukte der Formel III kann in Analogie zur Herstellung entsprechender Verbindungen der Formel I erfolgen.

Die Ausgangsprodukte der Formeln IV, V und VI sind bekannt oder nach an sich bekannten und jedem Fachmann geläufigen Methoden leicht herstellbar; zudem enthalten manche der nachstehenden Beispiele detaillierte Angaben über die Herstellung bestimmter Verbindungen der Formel V.

Die Ausgangsprodukte der Formel VII können beispielsweise dadurch hergestellt werden, dass man eine Verbindung der Formel

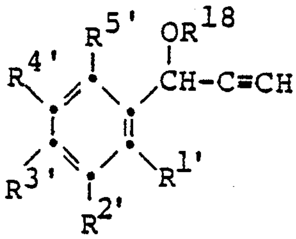

VIII

worin $R^{1'}$, $R^{2'}$, $R^{3'}$, $R^{4'}$, $R^{5'}$ und $R^{18}$ obige Bedeutung besitzen,
in Gegenwart einer starken Base, wie n-Butyllithium oder dergleichen, in einem unter den Reaktionsbedingungen inerten organischen Lösungsmittel oder Lösungsmittelgemisch, wie Aether/n-Hexan,

Tetrahydrofuran/n-Hexan oder dergleichen, mit einer Verbindung der Formel

X-$R^{6iv}$ IX

worin $R^{6iv}$ obige Bedeutung besitzt und X eine Abgangsgruppe (insbesondere Chlor) bedeutet, umsetzt, beispielsweise also mit Chlorameisensäurebutylester, N,N-Dimethylcarbamoylchlorid oder dergleichen.

Die Herstellung der Ausgangsprodukte der Formel VIII erfolgt nach an sich bekannten und jedem Fachmann geläufigen Methoden aus entsprechenden Verbindungen der Formel

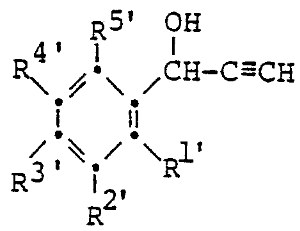

X

worin $R^{1'}$, $R^{2'}$, $R^{3'}$, $R^{4'}$ und $R^{5'}$ obige Bedeutung besitzen, durch Einführung der gewünschten Schutzgruppe, beispielsweise also mittels Trimethylsilylchlorid in Gegenwart von n-Butyllithium in Aether/n-Hexan, mittels t-Butyldimethylsilylchlorid in Gegenwart von 1,8-Diazabicyclo[5.4.0]undec-7-en in Methylenchlorid usw.

Die Ausgangsprodukte der Formeln IX und X sind bekannt oder nach an sich bekannten und jedem Fachmann geläufigen Methoden leicht herstellbar.

Wie oben erwähnt, besitzen die Verbindungen der allgemeinen Formeln I und II sowie pharmazeutisch verwendbare Salze von Verbindungen der Formel I wertvolle pharmakodynamische Eigenschaften.

Repräsentative Verbindungen der Formeln I und II wurden auf ihre mucosaprotektiven und magensäuresekretionshemmenden Eigenschaften sowie auf ihre Toxizität untersucht.

Zur Bestimmung der muscosa-protektiven Eigenschaft wurde die nachstehend beschriebene Versuchsanordnung verwendet:

Orale Verabreichung von absolutem Aethanol an männliche Ratten in einer Dosis von 1 ml pro Ratte führt innerhalb 1 Stunde zu blutigen Läsionen der Magenschleimhaut. Verschiedene Dosen der zu prüfenden Substanzen (suspendiert in 0,125% Carboxymethylcellulose) oder das Vehikel allein (Kontrolle) werden den Ratten oral (1 ml pro Ratte) 30 Minuten vor der Behandlung mit Aethanol verabreicht. Eine Stunde nach der Verabreichung des Aethanols tötet man die Tiere, untersucht deren Mägen auf das Vorhandensein von Läsionen und ermittelt die Anzahl und die gesamte Ausdehnung solcher Läsionen. Als $ID_{50}$ bezeichnet man diejenige Dosis einer Testsubstanz, welche die Anzahl der Läsionen im Vergleich zur Kontrollgruppe um 50% reduziert.

Zur Bestimmung der magensäuresekretionshemmenden Wirkung wurde die nachstehend beschriebene Versuchsanordnung verwendet:

Männlichen Ratten wird unter leichter Aethernarkose gemäss Shay et al. [Gastroenterology 5, 43 (1945)] der Pylorus ligiert. Die zu prüfenden Substanzen, suspendiert in 0,5% Carboxymethylcellulose, werden intraduodenal verabreicht; Kontrolltiere werden lediglich mit dem Vehikel behandelt. Fünf Stunden nach der Ligation tötet man die Tiere, bestimmt das Volumen und die Azidität ihres Magensaftes und vergleicht die erhaltenen Werte mit denjenigen von Kontrolltieren. Als $ID_{50}$ bezeichnet man diejenige Dosis einer Testsubstanz, welche bei den behandelten Tieren im Vergleich zu den Kontrolltieren eine 50%ige Verminderung der Sekretion bewirkt.

In der nachfolgenden Tabelle werden für eine Reihe repräsentativer Verbindungen der Formel I und für eine Verbindung der Formel II die Resultate der Prüfung auf ihre mucosa-protektive Wirkung ("Aethanol-Test") und auf ihre magensäuresekretionshemmende Wirkung wiedergegeben. Ausserdem enthält diese Tabelle Angaben über die akute Toxizität ($DL_{50}$ bei einmaliger oraler Verabreichung an Mäuse).

| Verbindung | Aethanol-Test, ID 50 mg/kg p.o. | Magensäuresekretionshemmung, ID 50 mg/kg i.d. | Toxizität, DL 50 mg/kg p.o. |
|---|---|---|---|
| A | 1,2 | 42 | 625-1250 |
| B | 1,9 | 18 | 156- 312 |
| C | 1,4 | - | 625-1250 |
| D | 2,1 | - | 625-1250 |
| E | 1,2 | - | 312- 625 |
| F | 2,9 | - | >5000 |
| G | 2,1 | - | 1000-2000 |
| H | 2,2 | - | >5000 |
| I | 1,9 | - | 625-1250 |
| J | 2,3 | > 100 | 1250-2500 |
| K | 3,4 | - | 500-1000 |
| L | 4,1 | 56 | 1000-2000 |
| M | 4,2 | - | 1250-2500 |
| N | 4,2 | > 100 | >5000 |
| O | 2,8 | 28 | 312- 625 |
| P | 0,9 | - | 80- 156 |
| Q | 1,5 | - | 80- 156 |
| R | 1;4 | - | 80- 156 |
| S | 1,2 | - | 80- 156 |
| T | 1,2 | - | 40- 80 |
| U | 1,1 | - | 80- 156 |
| V | 1,2 | 3,1 | 156- 312 |
| W | 0,8 | 9 | 312- 625 |
| X | 0,8 | 4,0 | 156- 312 |
| Y | 1,0 | 2,7 | 156- 312 |
| Aa | 1,4 | - | 312- 625 |
| Bb | 4,0 | - | 625-1250 |

A = 3-[3,4-(Methylendioxy)benzoyl]propiolsäure
B = 4-Hydroxy-1-(3,4,5-trimethoxyphenyl)-2-butin-1-on
C = 3-(4-Methoxybenzoyl)propiolsäure
D = 3-(2,3,4-Trimethoxybenzoyl)propiolsäuremethylester
E = 3-(4-Hydroxybenzoyl)propiolsäuremethylester
F = 3-(3,4,5-Trimethoxybenzoyl)propiolsäure-[2-(2-methoxyäthoxy)äthylester
G = 3-(4-Methoxybenzoyl)propiolsäure-[2-(2-methoxyäthoxy)äthyl]ester
H = 3-(2,5-Dimethoxybenzoyl)propiolsäuremethylester
I = 3-(2,5-Dimethoxybenzoyl)propiolsäure
J = 3-[3,4-(Methylendioxy)benzoyl]propiolsäure-[2-(2-methoxyäthoxy)äthyl]ester

K = 3-(2,3,4-Trimethoxybenzoyl)propiolsäure
L = 3-(3,4,5-Trimethoxybenzoyl)propiolsäure
M = 3-(4-Fluorbenzoyl)propiolsäuremethylester
N = 3-(3,4,5-Trimethoxybenzoyl)propiolsäuremethylester
O = N,N-Dimethyl-3-(3,4,5-trimethoxybenzoyl)propiolamid
P = 1-(4-Fluorphenyl)-4-hydroxy-2-butin-1-on
Q = 4-Hydroxy-1-(4-methylphenyl)-2-butin-1-on
R = 4-(1-Aethoxyäthoxy)-1-(4-fluorphenyl)-2-butin-1-on
S = 1-(4-Chlorphenyl)-4-hydroxy-2-butin-1-on
T = 4-Hydroxy-1-(4-methoxyphenyl)-2-butin-1-on
U = 4-Hydroxy-1-[3,4-(methylendioxy)phenyl]-2-butin-1-on
V = 4-Hydroxy-1-(3,4,5-trimethoxyphenyl)-2-pentin-1-on
W = 4-(1-Aethoxyäthoxy)-1-(2,3,4-trimethoxyphenyl)-2-butin-1-on
X = 4-Hydroxy-1-(2,3,4-trimethoxyphenyl)-2-butin-1-on
Y = 4-Hydroxy-1-[4-(methylthio)phenyl]-2-butin-1-on
Aa = 4-Hydroxy-4-[3,4-methylendioxy)phenyl]-2-butinsäuremethylester
Bb = 4-Hydroxy-4-(3,4,5-trimethoxyphenyl)-2-butinsäure-[2-(2-methoxyäthoxy)äthyl]ester

Die Verbindungen der Formeln I und II und die pharmazeutisch verwendbaren Salze von Verbindungen der Formel I können als Heilmittel, z.B. in Form pharmazeutischer Präparate Verwendung finden. In erster Linie kommt orale Verabreichung in Form von festen pharmazeutischen Präparaten, wie Tabletten, Lacktabletten, Dragées, Hartgelatinekapseln und Weichgelatinekapseln in Frage. Orale Verabreichung in Form flüssiger pharmazeutischer Präparate, wie Lösungen, Emulsionen und Suspensionen, rektale Verabreichung, z.B. in Form von Suppositorien, oder parenterale Verabreichung, z.B. in Form von Injektionslösungen, sind ebenfalls in Betracht zu ziehen.

Arzneimittel, enthaltend eine Verbindung der Formel I oder II oder ein pharmazeutisch verwendbares Salz einer Verbindung der Formel I, sind ebenfalls Gegenstand der vorliegenden Erfindung. Die Herstellung derartiger Arzneimittel kann dadurch erfolgen, dass man eine oder mehrere der Verbindungen der Formeln I oder II oder der pharmazeutisch verwendbaren Salze der Verbindungen der Formel I und erwünschtenfalls einen oder mehrere andere therapeutische Wirkstoffe zusammen mit einem oder mehreren therapeutisch inerten Excipientien in eine galenische Darreichungsform bringt.

Zur Herstellung von Tabletten, Lacktabletten, Dragées und Hartgelatinekapseln können die Verbindungen der Formeln I und II und die pharmazeutisch verwendbaren Salze von Verbindungen der Formel I mit pharmazeutisch inerten, anorganischen oder organischen Excipientien verarbeitet werden. Als solche Excipientien kann man z.B. für Tabletten, Dragées und Hartgelatinekapseln Lactose, Maisstärke oder Derivate davon, Talk, Stearinsäure oder deren Salze etc. verwenden. Zur Herstellung von magensaftresistenten pharmazeutischen Präparaten ist noch ein magensaftresistenter Lack aufzubringen, welcher z.B. aus Hydroxypropylmethylcellulosephthalat bestehen kann.

Für Weichgelatinekapseln eignen sich als Excipientien z.B. vegetabile Oele, Wachse, Fette, halbfeste und flüssige Polyole etc.

Zur Herstellung von Lösungen und Sirupen eignen sich als Excipientien z.B. Wasser, Polyole, Saccharose, Invertzucker, Glucose und dgl.

Für Suppositorien eignen sich als Excipientien z.B. natürliche oder gehärtete Oele, Wachse, Fette, halbflüssige oder flüssige Polyole und dgl.

Für Injektionslösungen eignen sich als Excipientien z.B. Wasser, Alkohole, Polyole, Glycerin, vegetabile Oele etc.

Die pharmazeutischen Präparate können daneben noch Konservierungsmittel, Lösevermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Erfindungsgemäss kann man die Verbindungen der Formeln I und II und die pharmazeutisch verwendbaren Salze von Verbindungen der Formel I bei der Bekämpfung bzw. Verhütung von Krankheiten verwenden, beispielsweise bei der Bekämpfung bzw. Verhütung von Ulcus ventriculi und/oder duodeni. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 30-400 mg und bei intravenöser Verabreichung eine Tagesdosis von etwa 1-50 mg angemessen sein.

Gegenstand der Erfindung ist auch die Verwendung der Verbindungen der Formeln I und II und der pharmazeutisch verwendbaren Salze von Verbindungen der Formel I zur Herstellung von Arzneimitteln

gegen Ulcus ventriculi und/oder duodeni.

In den nachfolgenden Beispielen, welche die vorliegende Erfindung illustrieren, ihren Umfang aber in keiner Weise beschränken sollen, sind sämtliche Temperaturen in Celsiusgraden angegeben.

Beispiel 1

a) Eine Lösung von 20 g (0,36 Mol) 2-Propin-1-ol in 637 ml (6,66 Mol) Aethylvinyläther wird bei 0° unter Argon mit 1,27 ml (16,7 mMol) Trifluoressigsäure versetzt, und anschliessend wird 65 Stunden bei Raumtemperatur gerührt. Man gibt 1,3 g Natriumcarbonat zu, rührt noch 30 Minuten bei Raumtemperatur und engt das Reaktionsgemisch am Rotationsverdampfer ein. Destillation des Rückstandes unter vermindertem Druck liefert 1-(1-Aethoxyäthoxy)-2-propin vom Siedepunkt 55°/30 mmHg.
MS: 127 (M-H) m/e.

b) Eine Lösung von 8 g (62,4 mMol) 1-(1-Aethoxyäthoxy)-2-propin in 135 ml Tetrahydrofuran wird unter Argon bei -78° mit 39 ml n-Butyllithium (1,6M in Hexan) versetzt. Man rührt das Gemisch 30 Minuten bei -40° und gibt dann innerhalb von 10 Minuten eine Lösung von 12,3 g (62,4 mMol) 2,3,4-Trimethoxybenzaldehyd in 53 ml Tetrahydrofuran zu. Das Reaktionsgemisch wird auf 0° erwärmt, noch 1 Stunde bei 0° gerührt und dann mit 100 ml gesättigter Ammoniumchlorid-Lösung versetzt. Die Wasserphase wird zweimal mit Essigester extrahiert; die vereinigten organischen Phasen werden nacheinander mit gesättigter Kochsalzlösung und mit Wasser gewaschen, über Magnesiumsulfat getrocknet und eingeengt. Der Rückstand wird durch Flash-Chromatographie an 600 g Silicagel (Elutionsmittel Aether/Hexan 1:1) gereinigt. Man erhält 4-(1-Aethoxyäthoxy)-1-(2,3,4-trimethoxyphenyl)-2-butin-1-ol als gelbes Oel.
MS: 324 ($M^+$) m/e
IR (Film): 1600, 1495, 1467, 1282, 1096 $cm^{-1}$

c) Eine Lösung von 12 g (37 mMol) 4-(1-Aethoxyäthoxy)-1-(2,3,4-trimethoxyphenyl)-2-butin-1-ol in 50 ml Methylenchlorid wird bei 0° zu einer Suspension von 95,3 g (1,1 Mol) Mangandioxid in 150 ml Methylenchlorid getropft. Das Reaktionsgemisch wird 15 Minuten bei 0° gerührt, über Magnesiumsulfat filtriert und eingeengt. Chromatographie des Rückstandes über 300 g Silicagel (Elutionsmittel Aether/Hexan 1:1) liefert 4-(1-Aethoxyäthoxy)-1-(2,3,4-trimethoxyphenyl)-2-butin-1-on als gelbes Oel.
MS: 322 ($M^+$) m/e
IR (Film): 2214, 1625, 1586, 1494, 1289 $cm^{-1}$

Beispiel 2

a) Eine Lösung von 6 g (47 mMol) 1-(1-Aethoxyäthoxy)-2-propin in 100 ml Tetrahydrofuran wird unter Argon bei -78° mit 29,2 ml n-Butyllithium (1,6M in Hexan) versetzt. Man rührt das Gemisch 30 Minuten bei -40° und gibt dann innerhalb von 10 Minuten eine Lösung von 6,6 g (47 mMol) 4-Chlorbenzaldehyd in 41 ml Tetrahydrofuran zu. Das Reaktionsgemisch wird auf 0° erwärmt, noch 1 Stunde bei 0° gerührt und dann mit 80 ml gesättigter Ammoniumchlorid-Lösung versetzt. Die Wasserphase wird zweimal mit Essigester extrahiert; die vereinigten organischen Phasen werden nacheinander mit gesättigter Kochsalzlösung und mit Wasser gewaschen, über Magnesiumsulfat getrocknet und eingeengt. Der Rückstand wird durch Flash-Chromatographie an 500 g Silicagel (Elutionsmittel Aether/Hexan 1:1) gereinigt. Man erhält 4-(1-Aethoxyäthoxy)-1-(4-chlorphenyl)-2-butin-1-ol als Oel.
MS: 253 ($M-CH_3$) m/e
IR (Film): 3405, 1596, 1490, 1128, 1088 $cm^{-1}$

b) Eine Lösung von 7,1 g (26,4 mMol) 4-(1-Aethoxyäthoxy)-1-(4-chlorphenyl)-2-butin-1-ol in 40 ml Methylenchlorid wird bei 0° zu einer Suspension von 68 g (0,78 Mol) Mangandioxid in 110 ml Methylenchlorid getropft. Das Reaktionsgemisch wird 10 Minuten bei 0° gerührt, über Magnesiumsulfat filtriert und eingeengt. Chromatographie des Rückstandes an 300 g Silicagel (Elutionsmittel Aether/Hexan 1:1) liefert 4-(1-Aethoxyäthoxy)-1-(4-chlorphenyl)-2-butin-1-on als Oel.
MS: 251 ($M-CH_3$) m/e

Beispiel 3

a) Eine Lösung von 5 g (33 mMol) 1-(1-Aethoxyäthoxy)-2-propin in 84 ml Tetrahydrofuran wird unter Argon bei -78° mit 24,4 ml n-Butyllithium (1,6M in Hexan) versetzt. Man rührt das Gemisch 30 Minuten bei -40° und gibt dann innerhalb von 10 Minuten eine Lösung von 4,6 ml (33 mMol) 4-Methylbenzaldehyd in 34 ml Tetrahydrofuran zu. Das Reaktionsgemisch wird auf 0° erwärmt, noch 1 Stunde bei 0° gerührt und dann mit 60 ml gesättigter AmmoniumchloridLösung versetzt. Die Wasserphase wird zweimal mit Aether extrahiert; die vereinigten organischen Phasen werden nacheinander mit gesättigter Kochsalzlösung und mit Wasser gewaschen, über Magnesiumsulfat getrocknet und eingeengt. Der Rückstand wird durch Chromatographie an 500 g Silicagel (Elutionsmittel Aether/Hexan 1:1) gereinigt. Man erhält 4-(1-Aethoxyäthoxy)-1-(4-methylphenyl)-2-butin-1-ol.
MS: 247 (M-H) m/e
IR (Film): 1512, 1128, 1086 $cm^{-1}$

b) Eine Lösung von 6,7 g (27 mMol) 4-(1-Aethoxyäthoxy)-1-(4-methylphenyl)-2-butin-2-ol in 40 ml Methylenchlorid wird bei 0° zu einer Suspension von 69 g (0,79 Mol) Mangandioxid in 110 ml Methylenchlorid getropft. Das Reaktionsgemisch wird 10 Minuten bei 0° gerührt, über Magnesiumsulfat filtriert und eingeengt. Chromatographie des Rückstandes in 300 g Silicagel (Elutionsmittel Aether/Hexan 1:1) liefert 4-(1-Aethoxyäthoxy)-1-(4-methylphenyl)-2-butin-1-on als gelbes Oel.
MS: 231 ($M-CH_3$) m/e
IR (Film): 2228, 1646, 1605, 1287 $cm^{-1}$

Beispiel 4

a) Eine Lösung von 8 g (62,4 mMol) 1-(1-Aethoxyäthoxy)-2-propin in 134 ml Tetrahydrofuran wird unter Argon bei -78° mit 39 ml n-Butyllithium (1,6M in Hexan) versetzt. Man rührt das Gemisch 30 Minuten bei -40° und gibt dann innerhalb von 10 Minuten eine Lösung von 6,6 ml (62 mMol) 4-Fluorbenzaldehyd in 54 ml Tetrahydrofuran zu. Das Reaktionsgemisch wird auf 0° erwärmt, noch 1 Stunde bei 0° gerührt und dann mit 100 ml gesättigter Ammoniumchlorid-Lösung versetzt. Die Wasserphase wird zweimal mit Essigester extrahiert; die vereinigten organischen Phasen werden nacheinander mit gesättigter Kochsalzlösung und mit Wasser gewaschen, über Magnesiumsulfat getrocknet, und eingeengt. Der Rückstand wird durch Chromatographie an 600 g Silicagel (Elutionsmittel Aether/Hexan 1:1) gereinigt. Man erhält 4-(1-Aethoxyäthoxy)-1-(4-fluorphenyl)-2-butin-1-ol als Oel.
MS: 251 (M-H) m/e
IR (Film): 3408, 1604, 1508 $cm^{-1}$

b) Eine Lösung von 9,3 g (37 mMol) 4-(1-Aethoxyäthoxy)-1-(4-fluorphenyl)-2-butin-1-ol in 50 ml Methylenchlorid wird bei 0° zu einer Suspension von 95 g (1,1 Mol) Mangandioxid in 150 ml Methylenchlorid getropft. Das Reaktionsgemisch wird 10 Minuten bei 0° gerührt, über Magnesiumsulfat filtriert und eingeengt. Chromatographie des Rückstandes an 300 g Silicagel (Elutionsmittel Aether/Hexan 1:1) liefert 4-(1-Aethoxyäthoxy)-1-(4-fluorphenyl)-2-butin-1-on als Oel.
MS: 235 ($M-CH_3$) m/e
IR (Film): 2228, 1651, 1596, 1504 $cm^{-1}$

Beispiel 5

a) Eine Lösung von 6 g (47 mMol) 1-(1-Aethoxyäthoxy)-2-propin in 100 ml Tetrahydrofuran wird unter Argon bei -78° mit 29,2 ml n-Butyllithium (1,6M in Hexan) versetzt. Man rührt das Gemisch 30 Minuten bei -40° und gibt dann innerhalb von 10 Minuten eine Lösung von 5,7 ml (47 mMol) 4-Methoxybenzaldehyd in 41 ml Tetrahydrofuran zu. Das Reaktionsgemisch wird auf 0° erwärmt, noch 1 Stunde bei 0° gerührt und dann mit 80 ml gesättigter Ammoniumchlorid-Lösung versetzt. Die Wasserphase wird zweimal mit Essigester extrahiert; die vereinigten organischen Phasen werden nacheinander mit gesättigter Kochsalzlösung und mit Wasser gewaschen, über Magnesiumsulfat getrocknet und eingeengt. Der Rückstand wird durch Flash-Chromatographie an 500 g Silicagel (Elutionsmittel Aether/Hexan 1:1) gereinigt. Man erhält 4-(1-Aethoxyäthoxy)-1-(4-methoxyphenyl)-2-butin-1-ol als Oel.
MS: 264 ($M^+$) m/e
IR (Film): 3412, 1811, 1512, 1250 $cm^{-1}$

b) Eine Lösung von 7,6 g (29 mMol) 4-(1-Aethoxyäthoxy)-1-(4-methoxyphenyl)-2-butin-1-ol in 56 ml

Methylenchlorid wird bei 0° zu einer Suspension von 74 g (0,85 Mol) Mangandioxid in 100 ml Methylenchlorid getropft. Das Reaktionsgemisch wird 10 Minuten bei 0° gerührt, über Magnesiumsulfat filtriert und eingeengt. Chromatographie des Rückstandes an 300 g Silicagel (Elutionsmittel Aether/Hexan 1:1) liefert 4-(1-Aethoxyäthoxy)-1-(4-methoxyphenyl)-2-butin-1-on als Oel.
MS: 247 ($M-CH_3$) m/e
IR (Film): 2229, 1642, 1598, 1509, 1259 $cm^{-1}$

Beispiel 6

a) Eine Lösung von 8 g (62,4 mMol) 1-(1-Aethoxyäthoxy)-2-propin in 134 ml Tetrahyrofuran wird unter Argon bei -78° mit 39 ml n-Butyllithium (1,6M in Hexan) versetzt. Man rührt das Gemisch 30 Minuten bei -40° und gibt dann innerhalb von 10 Minuten eine Lösung von 9,4 ml (62,4 mMol) 3,4-Methylendioxybenzaldehyd in 54 ml Tetrahydrofuran zu. Das Reaktionsgemisch wird auf 0° erwärmt, noch 1 Stunde bei 0° gerührt und dann mit 100 ml gesättigter Ammoniumchlorid-Lösung versetzt. Die Wasserphase wird zweimal mit Essigester extrahiert; die vereinigten organischen Phasen werden nacheinander mit gesättigter Kochsalzlösung und mit Wasser gewaschen, über Magnesiumsulfat getrocknet und eingeengt. Der Rückstand wird durch Chromatographie an 500 g Silicagel (Elutionsmittel Aether/Hexan 1:1) gereinigt. Man erhält 4-(1-Aethoxyäthoxy)-1-[3,4-(methylendioxy)phenyl]-2-butin-1-ol als Oel.
MS: 278 ($M^+$) m/e
IR (Film): 3410, 1487, 1448, 1247 $cm^{-1}$

b) Eine Lösung von 10,9 g (39,2 mMol) 4-(1-Aethoxyäthoxy)-1-[3,4-(methylendioxy)phenyl]-2-butin-1-ol in 63 ml Methylenchlorid wird bei 0° zu einer Suspension von 101 g (1,16 Mol) Mangandioxid in 150 ml Methylenchlorid getropft. Das Reaktionsgemisch wird 10 Minuten bei 0° gerührt, über Magnesiumsulfat filtriert und eingeengt. Chromatographie des Rückstandes an 300 g Silicagel (Elutionsmittel Aether/Hexan 1:1) liefert 4-(1-Aethoxyäthoxy)-1-[3,4-(methylendioxy)phenyl]-2-butin-1-on als Oel.
MS: 276 ($M^+$) m/e
IR (Film): 2230, 1640, 1602, 1289 $cm^{-1}$

Beispiel 7

a) Eine Lösung von 16 g (124,8 mMol) 1-(1-Aethoxyäthoxy)-2-propin in 270 ml Tetrahydrofuran wird unter Argon bei -78° mit 78 ml n-Butyllithium (1,6M in Hexan) versetzt. Man rührt das Gemisch 30 Minuten bei -40° und gibt dann innerhalb von 10 Minuten eine Lösung von 7,62 g (62,4 mMol) 4-Hydroxybenzaldehyd in 53 ml Tetrahydrofuran zu. Das Reaktionsgemisch wird auf 0° erwärmt, noch 1 Stunde bei 0° gerührt und dann mit 100 ml gesättigter Ammoniumchlorid-Lösung versetzt. Die Wasserphase wird zweimal mit Essigester extrahiert; die vereinigten organischen Phasen werden nacheinander mit gesättigter Kochsalzlösung und mit Wasser gewaschen, über Magnesiumsulfat getrocknet und eingeengt. Der Rückstand wird durch Flash-Chromatographie an 600 g Silicagel (Elutionsmittel Aether/Hexan 1:1) gereinigt. Man erhält 4-(1-Aethoxyäthoxy)-1-(4-hydroxyphenyl)-2-butin-1-ol als Oel.
MS: 250 ($M^+$) m/e
IR (Film): 3268, 1614, 1516, 1276 $cm^{-1}$

b) Eine Lösung von 8,6 g (34,4 mMol) 4-(1-Aethoxyäthoxy)-1-(4-hydroxyphenyl)-2-butin-1-ol in 50 ml Methylenchlorid wird bei 0° zu einer Suspension von 89 g (1,02 Mol) Mangandioxid in 150 ml Methylenchlorid getropft. Das Reaktionsgemisch wird 15 Minuten bei 0° gerührt, über Magnesiumsulfat filtriert und eingeengt. Flash-Chromatographie des Rückstandes an 200 g Silicagel (Elutionsmittel Aether/Hexan 1:1) liefert 4-(1-Aethoxyäthoxy)-1-(4-hydroxyphenyl)-2-butin-1-on als Oel.
MS: 233 ($M-CH_3$) m/e
IR (Film): 3267, 2232, 1629, 1577, 1512 $cm^{-1}$

Beispiel 8

a) Eine Lösung von 8 g (62,4 mMol) 1-(1-Aethoxyäthoxy)-2-propin in 135 ml Tetrahyrofuran wird unter Argon bei -78° mit 39 ml n-Butyllithium (1,6M in Hexan) versetzt. Man rührt das Gemisch 30 Minuten bei -40° und gibt dann innerhalb von 10 Minuten eine Lösung von 8,11 ml (62,4 mMol) 4-(Methylthio)-benzaldehyd in 53 ml Tetrahydrofuran zu. Das Reaktionsgemisch wird auf 0° erwärmt, noch 1 Stunde bei

0° gerührt und dann mit 100 ml gesättigter Ammoniumchlorid-Lösung versetzt. Die Wasserphase wird zweimal mit Essigester extrahiert; die vereinigten organischen Phasen werden nacheinander mit gesättigter Kochsalzlösung und mit Wasser gewaschen, über Magnesiumsulfat getrocknet und eingeengt. Der Rückstand wird durch Flash-Chromatographie an 600 g Silicagel (Elutionsmittel Aether/Hexan 1:1) gereinigt. Man erhält 4-(1-Aethoxyäthoxy)-1-[4-(methylthio)phenyl]-2-butin-1-ol als Oel.
MS: 280 ($M^+$) m/e
IR (Film): 3407, 2116, 1598, 1492 $cm^{-1}$

b) Eine Lösung von 10,9 g (38,9 mMol) 4-(1-Aethoxyäthoxy)-1-[4-(methylthio)phenyl]-2-butin-1-ol in 50 ml Methylenchlorid wird bei 0° zu einer Suspension von 100 g (1,15 Mol) Mangandioxid in 170 ml Methylenchlorid getropft. Das Reaktionsgemisch wird 15 Minuten bei 0° gerührt, über Magnesiumsulfat filtriert und eingeengt. Flash-Chromatographie des Rückstandes an 200 g Silicagel (Elutionsmittel Aether/Hexan 1:1) liefert 4-(1-Aethoxyäthoxy)-1-[4-(methylthio)phenyl]-2-butin-1-on als Oel.
MS: 278 ($M^+$) m/e
IR (Film): 2230, 1640, 1588, 1105 $cm^{-1}$

Beispiel 9

Eine Lösung von 8,3 g (27,6 mMol) 4-(1-Aethoxyäthoxy)-1-(2,3,4-trimethoxyphenyl)-2-butin-1-on in 95 ml Tetrahydrofuran wird bei Raumtemperatur mit 27,5 ml 2N Salzsäure versetzt, worauf 30 Minuten gerührt wird. Das Reaktionsgemisch wird dann zweimal mit Essigester extrahiert; die vereinigten organischen Phasen werden nacheinander mit gesättigter Natriumcarbonat-Lösung und mit Wasser gewaschen, über Magnesiumsulfat getrocknet und eingeengt. Kristallisation des Rückstandes aus Essigester/Hexan liefert 4-Hydroxy-1-(2,3,4-trimethoxyphenyl)-2-butin-1-on vom Schmelzpunkt 82-83°.

Beispiel 10

Eine Lösung von 6,7 g (25 mMol) 4-(1-Aethoxyäthoxy)-1-(4-chlorphenyl)-2-butin-1-on in 90 ml Tetrahydrofuran wird bei Raumtemperatur mit 25 ml 2N Salzsäure versetzt, worauf 30 Minuten gerührt wird. Das Reaktionsgemisch wird dann zweimal mit Essigester extrahiert; die vereinigten organischen Phasen werden nacheinander mit gesättigter Natriumcarbonat-Lösung und mit Wasser gewaschen, über Magnesiumsulfat getrocknet und eingeengt. Kristallisation des Rückstandes aus Essigester/Hexan liefert 1-(4-Chlorphenyl)-4-hydroxy-2-butin-1-on vom Schmelzpunkt 74°.

Beispiel 11

Eine Lösung von 5,7 g (23 mMol) 4-(1-Aethoxyäthoxy)-1-(4-methylphenyl)-2-butin-1-on in 80 ml Tetrahydrofuran wird bei Raumtemperatur mit 23 ml 2N Salzsäure versetzt, worauf 30 Minuten gerührt wird. Das Reaktionsgemisch wird dann zweimal mit Essigester extrahiert; die vereinigten organischen Phasen werden nacheinander mit gesättigter Natriumcarbonat-Lösung und mit Wasser gewaschen, über Magnesiumsulfat getrocknet und eingeengt. Kristallisation des Rückstandes aus Essigester/Hexan liefert 4-Hydroxy-1-(4-methylphenyl)-2-butin-1-on vom Schmelzpunkt 74°.

Beispiel 12

Eine Lösung von 7,3 g (29 mMol) 4-(1-Aethoxyäthoxy)-1-(4-fluorphenyl)-2-butin-1-on in 100 ml Tetrahydrofuran wird bei Raumtemperatur mit 29 ml 2N Salzsäure versetzt, worauf 30 Minuten gerührt wird. Das Reaktionsgemisch wird dann zweimal mit Essigester extrahiert; die vereinigten organischen Phasen werden nacheinander mit gesättigter Natriumcarbonat-Lösung und mit Wasser gewaschen, über Magnesiumsulfat getrocknet und eingeengt. Kristallisation des Rückstandes aus Essigester/Hexan liefert 1-(4-Fluorphenyl)-4-hydroxy-2-butin-1-on vom Schmelzpunkt 63°.

Beispiel 13

Eine Lösung von 7,3 g (27,8 mMol) 4-(1-Aethoxyäthoxy)-1-(4-methoxyphenyl)-2-butin-1-on in 95 ml Tetrahydrofuran wird bei Raumtemperatur mit 28 ml 2N Salzsäure versetzt, worauf 30 Minuten gerührt wird. Das Reaktionsgemisch wird dann zweimal mit Essigester extrahiert; die vereinigten organischen Phasen werden nacheinander mit gesättigter Natriumcarbonat-Lösung und mit Wasser gewaschen, über Magnesiumsulfat getrocknet und eingeengt. Kristallisation des Rückstandes aus Methylenchlorid/Hexan liefert 4-Hydroxy-1-(4-methoxyphenyl)-2-butin-1-on vom Schmelzpunkt 84-85°.

Beispiel 14

Eine Lösung von 5,2 g (18,8 mMol) 4-(1-Aethoxyäthoxy)-1-[3,4-(methylendioxy)phenyl]-2-butin-1-on in 65 ml Tetrahydrofuran wird bei Raumtemperatur mit 18,7 ml 2N Salzsäure versetzt, worauf 30 Minuten gerührt wird. Das Reaktionsgemisch wird dann zweimal mit Essigester extrahiert; die vereinigten organischen Phasen werden nacheinander mit gesättigter Natriumcarbonat-Lösung und mit Wasser gewaschen, über Magnesiumsulfat getrocknet und eingeengt. Kristallisation des Rückstandes aus Essigester/Hexan liefert 4-Hydroxy-1-[3,4-(methylendioxy)phenyl]-2-butin-1-on vom Schmelzpunkt 91-93°.

Beispiel 15

Eine Lösung von 10,2 g (36,6 mMol) 4-(1-Aethoxyäthoxy)-1-[4-methylthio)phenyl]-2-butin-1-on in 126 ml Tetrahydrofuran wird bei Raumtemperatur mit 36,5 ml 2N Salzsäure versetzt, worauf 30 Minuten gerührt wird. Das Reaktionsgemisch wird dann zweimal mit Essigester extrahiert; die vereinigten organischen Phasen werden nacheinander mit gesättigter Natriumcarbonat-Lösung und mit Wasser gewaschen, über Magnesiumsulfat getrocknet und eingeengt. Chromatogra phie des Rückstandes an 350 g Silicagel (Elutionsmittel Methylenchlorid/Essigester 9:1) und anschliessende Kristallisation aus Essigester/Hexan liefert 4-Hydroxy-1-[4-(methylthio)phenyl]-2-butin-1-on vom Schmelzpunkt 73-74°.

Beispiel 16

Zu einer Lösung von 10,0 g (71,3 mMol) 3-[(Tetrahydro-2H-pyran-2-yl)oxy]-1-propin in 150 ml absolutem Tetrahydrofuran werden bei -78° 44,6 ml n-Butyllithium-Lösung (1,6M in Hexan) zugegeben. Das Reaktionsgemisch wird 30 Minuten bei -40° gerührt und dann mit 14,0 g (71,3 mMol) 3,4,5-Trimethoxybenzaldehyd in 50 ml Tetrahydrofuran versetzt. Das Reaktionsgemisch wird anschliessend 1 Stunde bei 0° gerührt und dann mit 100 ml gesättigter Ammoniumchlorid-Lösung und 200 ml Aether versetzt. Die Wasserphase wird zweimal mit Aether extrahiert, worauf man die vereinigten organischen Phasen über Magnesiumsulfat trocknet und das Lösungsmittel abdestilliert. Der Rückstand wird in 150 ml Methylenchlorid gelöst und bei 0° zu einer Suspension von 186 g Mangandioxid in 300 ml Methylenchlorid getropft. Das Reaktionsgemisch wird 30 Minuten bei Raumtemperatur gerührt, über Magnesiumsulfat filtriert und eingedampft. Der Rückstand wird in 300 ml Tetrahydrofuran/Aethanol (1:1) gelöst und mit 2,4 g Pyridinium-p-toluolsulfonat versetzt. Man rührt während 24 Stunden bei Raumtemperatur, gibt dann 100 ml Wasser und 200 ml Aether zu, extrahiert die wässrige Phase zweimal mit Aether, trocknet die vereinigten organischen Phasen über Magnesiumsulfat und destilliert das Lösungsmittel ab. Kristallisation aus Essigester/Hexan liefert 4-Hydroxy-1-(3,4,5-trimethoxyphenyl)-2-butin-1-on vom Schmelzpunkt 104-105°.

Beispiel 17

a) Eine Lösung von 3,4 ml (40 mMol) Propiolsäuremethylester in 30 ml Tetrahydrofuran wird unter Argon bei -78° mit 25 ml n-Butyllithium (1,6M in Hexan) versetzt. Man rührt das Gemisch 10 Minuten bei -78° und gibt dann innerhalb von 10 Minuten eine Lösung von 7,85 g (40 mMol) 2,3,4-Trimethoxybenzaldehyd in 40 ml Tetrahydrofuran zu. Das Reaktionsgemisch wird auf Raumtemperatur gebracht, 15 Minuten nachgerührt und mit 60 ml gesättigter Ammoniumchlorid-Lösung versetzt. Die Wasserphase wird zweimal mit Aether extrahiert; die vereinigten organischen Phasen werden nacheinander mit gesättigter Kochsalzlösung und mit Wasser gewaschten, über Natriumsulfat getrocknet und eingeengt.

Der Rückstand wird durch Flash-Chromatographie an 500 g Silicagel (Elutionsmittel Methylenchlorid/Essigester 9:1) gereinigt. Man erhält 4-Hydroxy-4-(2,3,4-trimethoxyphenyl)-2-butinsäuremethylester als rotes Oel.
MS: 280 ($M^+$) m/e

b) Eine Lösung von 7 g (25 mMol) 4-Hydroxy-4-(2,3,4-trimethoxyphenyl)-2-butinsäuremethylester in 40 ml Methylenchlorid wird bei 0° zu einer Suspension von 62 g (0,71 Mol) Mangandioxid in 140 ml Methylenchlorid getropft. Das Reaktionsgemisch wird 1 Stunde bei 0° gerührt, über Magnesiumsulfat filtriert und eingeengt. Kristallisation des Rückstandes aus Essigester/Hexan liefert 3-(2.3.4-Trimethoxybenzoyl)-propiolsäuremethylester vom Schmelzpunkt 74°.

Beispiel 18

a) Zu einer Lösung von 9,6 ml (0,115 Mol) Propiolsäuremethylester in 195 ml Tetrahydrofuran/Aether/Hexan (4:2:1) werden bei -110° 71,6 ml n-Butyllithium-Lösung (1,6M in Hexan) zugegeben. Das Gemisch wird 20 Minuten bei -110° gerührt, worauf innerhalb von 20 Minuten 15,0 g (76,4 mMol) 3,4,5-Trimethoxybenzaldehyd in 15 ml Aether zugegeben werden. Das Reaktionsgemisch wird noch 90 Minuten bei -78° gerührt, dann langsam auf Raumtemperatur gebracht und hierauf mit 100 ml gesättigter Ammoniumchlorid-Lösung versetzt. Die Wasserphase wird zweimal mit Essigester extrahiert; die vereinigten organischen Phasen werden über Magnesiumsulfat getrocknet und eingedampft. Chromatographie an Kieselgel mit Essigester/Hexan (1:2) liefert 4-Hydroxy-4-(3,4,5-trimethoxyphenyl)-2-butinsäuremethylester.
MS: 280 ($M^+$) m/e
IR (Film): 2237(m), 1717(s), 1595(s), 1225(s), 1126(s), 1003(m).

b) Eine Lösung von 6,84 g (24,4 mMol) 4-Hydroxy-4-(3,4,5-trimethoxyphenyl)-2-butinsäuremethylester in 10 ml Methyllenchlorid wird bei 0° zu einer Suspension von 63,7 g (0,73 Mol) Mangandioxid in 130 ml Methylenchlorid getropft. Das Reaktionsgemisch wird 1 Stunde bei 0° gerührt, über Magnesiumsulfat filtriert und eingedampft. Kristallisation des Rückstands aus Essigester/Hexan liefert 3-(3,4,5-Trimethoxybenzoyl)-propiolsäuremethylester vom Schmelzpunkt 97°.

Beispiel 19

a) Eine Lösung von 5 ml (60 mMol) Propiolsäuremethylester in 60 ml Tetrahydrofuran wird unter Argon bei -78° mit 37,5 ml n-Butyllithium (1,6M in Hexan) versetzt. Man rührt das Gemisch 10 Minuten bei -78° und gibt dann innerhalb von 30 Minuten eine Lösung von 9 g (60 mMol) 3,4-Methylendioxybenzaldehyd in 60 ml Tetrahydrofuran zu. Das Reaktionsgemisch wird noch 20 Minuten bei -78° gerührt, dann auf Raumtemperatur gebracht und mit 80 ml gesättigter Ammoniumchlorid-Lösung versetzt. Die Wasserphase wird zweimal mit Aether extrahiert; die vereinigten organischen Phasen werden nacheinander mit gesättigter Kochsalzlösung und mit Wasser gewaschen, über Natriumsulfat getrocknet und eingeengt. Der Rückstand wird durch Flash-Chromtographie an 500 g Silicagel (Elutionsmittel Methylenchlorid/Essigester 9:1) gereinigt. Man erhält 4-Hydroxy-4-[3,4-(methylendioxy)phenyl] -2-butinsäuremethylester als braunes Oel.
MS: 234 ($M^+$) m/e

b) Eine Lösung von 7,3 g (31 mMol) 4-Hydroxy-4-[3,4-(methylendioxy)phenyl] -2-butinsäuremethylester in 80 ml Methylenchlorid wird bei 0° zu einer Suspension von 77 g (0,89 Mol) Mangandioxid in 150 ml Methylenchorid getropft. Das Reaktionsgemisch wird 30 Minuten bei 0° gerührt, über Magnesiumsulfat filtriert und eingeengt. Kristallisation des Rückstandes aus Aether/Hexan liefert 3-[3,4-(Methylendioxy)-benzoyl)propiolsäuremethylester vom Schmelzpunkt 95-97°.

Beispiel 20

a) Eine Lösung von 5 ml (60 mMol) Propiolsäuremethylester in 60 ml Tetrahydrofuran wird unter Argon bei -78° mit 37,5 ml n-Butyllithium (1,6M in Hexan) versetzt. Man rührt das Gemisch 10 Minuten bei -78° und gibt dann innerhalb von 30 Minuten eine Lösung von 7,3 ml (60 mMol) 4-Methoxybenzaldehyd in 70 ml Tetrahydrofuran zu. Das Reaktionsgemisch wird noch 20 Minuten bei -78° gerührt, dann auf Raumtemperatur gebracht und mit 80 ml gesättigter Ammoniumchlorid-Lösung versetzt. Die Wasserphase wird zweimal mit Aether extrahiert; die vereinigten organischen Phasen werden nacheinander mit gesättigter Kochsalzlösung und mit Wasser gewaschen, über Natriumsulfat getrocknet und eingeengt. Der Rückstand wird durch Flash-Chromtographie an 700 g Silicagel (Elutionsmittel Methylenchlorid/Essigester 9:1) gereinigt. Man erhält 4-Hydroxy-4-(4-methoxyphenyl)-2-butinsäuremethylester als rotes Oel.

MS: 220 ($M^+$) m/e

b) Eine Lösung von 7,5 g (34 mMol) 4-Hydroxy-4-(4-methoxyphenyl)-2-butinsäuremethylester in 100 ml Methylenchlorid wird bei 0° zu einer Suspension von 84,4 g (0,97 Mol) Mangandioxid in 150 ml Methylenchlorid getropft. Das Reaktionsgemisch wird 30 Minuten bei 0° gerührt, über Magnesiumsulfat filtriert und eingeengt. Kristallisation des Rückstandes aus Aether/Hexan liefert 3-(4-Methoxybenzoyl)-propiolsäuremethylester vom Schmelzpunkt 68°.

Beispiel 21

a) Eine Lösung von 2,16 ml (25,6 mMol) Propiolsäuremethylester in 30 ml Tetrahydrofuran wird unter Argon bei -78° mit 15,6 ml n-Butyllithium (1,6M in Hexan) versetzt. Man rührt das Gemisch 10 Minuten bei -78° und gibt dann innerhalb von 10 Minuten eine Lösung von 4,1 g (25,6 mMol) 5,6,7,8-Tetrahydronaphthyl-2-carboxaldehyd in 40 ml Tetrahydrofuran zu. Das Reaktionsgemisch wird noch 10 Minuten bei -78° gerührt, dann auf Raumtemperatur gebracht und mit 100 ml gesättigter Ammoniumchlorid-Lösung versetzt. Die Wasserphase wird zweimal mit Aether extrahiert; die vereinigten organischen Phasen werden nacheinander mit gesättigter Kochsalzlösung und mit Wasser gewaschen, über Natriumsulfat getrocknet und eingeengt. Der Rückstand wird durch Flash-Chromatographie an 500 g Silicagel (Elutionsmittel Methylenchlorid/Essigester 9:1) gereinigt. Man erhält 4-Hydroxy-4-[2-(5,6,7,8-tetrahydronaphthyl)] -2-butinsäuremethylester als rotes Oel.

MS: 244 ($M^+$), 229 (base peak) m/e

b) Eine Lösung von 3,3 g (13,5 mMol) 4-Hydroxy-4-[2-(5,6,7,8-tetrahydronaphthyl)] -2-butinsäuremethylester in 100 ml Methylenchlorid wird bei 0° zu einer Suspension von 35 g (0,4 Mol) Mangandioxid in 100 ml Methylenchlorid getropft. Das Reaktionsgemisch wird 20 Minuten bei 0° gerührt, über Magnesiumsulfat filtriert und eingeengt. Der Rückstand wird durch Flash-Chromatographie an 100 g Silicagel (Elutionsmittel Methylenchlorid/Essigester 9:1) gereinigt. Man erhält 3-[2-(5,6,7,8 - Tetrahydronaphthyl)carbonyl]propiolsäuremethylester als gelbes Oel.

MS: 242 ($M^+$), 131 (base peak) m/e

IR (Film): 1723, 1648, 1602, 1494, 1268, 1248 $cm^{-1}$

Beispiel 22

a) Eine Lösung von 2,8 ml (32 mMol) Propiolsäuremethylester in 60 ml Tetrahydrofuran wird unter Argon bei -78° mit 20 ml n-Butyllithium (1,6M in Hexan) versetzt. Man rührt das Gemisch 10 Minuten bei -78° und gibt dann innerhalb von 15 Minuten eine Lösung von 4,7 g (32 mMol) 5-Formylindan in 80 ml Tetrahydrofuran zu. Das Reaktionsgemisch wird noch 10 Minuten bei -78° gerührt, dann auf Raumtemperatur gebracht und mit 60 ml gesättigter Ammoniumchlorid-Lösung versetzt. Die Wasserphase wird zweimal mit Aether extrahiert; die vereinigten organischen Phasen werden nacheinander mit gesättigter Kochsalzlösung und mit Wasser gewaschen, über Natriumsulfat getrocknet und eingeengt. Der Rückstand wird durch Flash-Chromatographie an 500 g Silicagel (Elutionsmittel Methylenchlorid/Essigester 9:1) gereinigt. Man erhält 4-Hydroxy-4-(5-indanyl)-2-butinsäuremethylester als gelbes Oel.

MS: 230 ($M^+$), 215 (base peak) m/e

b) Eine Lösung von 6 g (26 mMol) 4-Hydroxy-4-(5-indanyl)-2-butinsäuremethylester in 200 ml Methylenchlorid wird bei 0° zu einer Suspension von 68 g (0,78 Mol) Mangandioxid in 200 ml Methylenchlorid getropft. Das Reaktionsgemisch wird 20 Minuten bei 0° gerührt, über Magnesiumsulfat filtriert und eingeengt. Der Rückstand wird durch Flash-Chromatographie an 500 g Silicagel (Elutionsmittel Methylenchlorid/Essigester 9:1) gereinigt. Man erhält 3-(5-Indanylcarbonyl)propiolsäuremethylester als gelbes Oel.

MS: 228 ($M^+$), 117 (base peak) m/e

Beispiel 23

a) Eine Lösung von 7,5 ml (90 mMol) Propiolsäuremethylester in 80 ml Tetrahydrofuran wird unter Argon bei -78° mit 56,3 ml n-Butyllithium (1,6M in Hexan) versetzt. Man rührt das Gemisch 10 Minuten bei -78° und gibt dann innerhalb von 40 Minuten eine Lösung von 8,4 ml (80 mMol) 4-Fluorbenzaldehyd in 80 ml Tetrahydrofuran zu. Das Reaktionsgemisch wird noch 20 Minuten bei -78° gerührt, dann auf Raumtemperatur gebracht und mit 80 ml gesättigter Ammoniumchlorid-Lösung versetzt. Die Wasserphase wird zweimal mit Aether extrahiert; die vereinigten organischen Phasen werden nacheinander mit gesättigter Kochsalzlösung und mit Wasser gewaschen, über Natriumsulfat getrocknet und eingeengt. Der Rückstand wird durch Flash-Chromatographie an 800 g Silicagel (Elutionsmittel Methylenchlorid/Essigester 9:1) gereinigt. Man erhält 4-(4-Fluorphenyl)-4-hydroxy-2-butinsäuremethylester als gelbes Oel.
MS: 208 ($M^+$), 123 (base peak) m/e

b) Eine Lösung von 7,5 g (36 mMol) 4-(4-Fluorphenyl)-4-hydroxy-2-butinsäuremethylester in 100 ml Methylenchlorid wird bei 0° zu einer Suspension von 89,3 g (1,03 Mol) Mangandioxid in 150 ml Methylenchlorid getropft. Das Reaktionsgemisch wird 30 Minuten bei 0° gerührt, über Magnesiumsulfat filtriert und eingeengt. Kristallisation des Rückstandes aus Aether/Hexan liefert 3-(4-Fluorbenzoyl)-propiolsäuremethylester vom Schmelzpunkt 62-63°.

Beispiel 24

a) Eine Lösung von 8,36 ml (0,1 Mol) Propiolsäuremethylester in 100 ml Tetrahydrofuran wird unter Argon bei -78° mit 68,8 ml n-Butyllithium (1,6M in Hexan) versetzt. Man rührt das Gemisch 10 Minuten bei -78° und gibt dann innerhalb von 40 Minuten eine Lösung von 14,06 g (0,1 Mol) 4-Chlorbenzaldehyd in 100 ml Tetrahydrofuran zu. Das Reaktionsgemisch wird noch 20 Minuten bei -78° gerührt, dann auf Raumtemperatur gebracht und mit 100 ml gesättigter Ammoniumchlorid-Lösung versetzt. Die Wasserphase wird zweimal mit Aether extrahiert; die vereinigten organischen Phasen werden nacheinander mit gesättigter Kochsalzlösung und mit Wasser gewaschen, über Natriumsulfat getrocknet und eingeengt. Der Rückstand wird durch Flash-Chromatographie an 1000 g Silicagel (Elutionsmittel Methylenchlorid/Essigester 9:1) gereinigt. Man erhält 4-(4-Chlorphenyl)-4-hydroxy-2-butinsäuremethylester als rotes Oel.
MS: 224 ($M^+$), 53 (base peak) m/e

b) Eine Lösung von 4 g (17,8 mMol) 4-(4-Chlorphenyl)-4-hydroxy-2-butinsäuremethylester in 50 ml Methylenchlorid wird bei 0° zu einer Suspension von 45 g (0,52 Mol) Mangandioxid in 100 ml Methylenchlorid getropft. Das Reaktionsgemisch wird 30 Minuten bei 0° gerührt, über Magnesiumsulfat filtriert und eingeengt. Kristallisation des Rückstandes aus Aether/Hexan liefert 3-(4-Chlorbenzoyl)-propiolsäuremethylester vom Schmelzpunkt 47-48°.

Beispiel 25

a) Eine Lösung von 8,4 ml (0,1 Mol) Propiolsäuremethylester in 100 ml Tetrahydrofuran wird unter Argon bei -78° mit 68,8 ml n-Butyllithium (1,6M in Hexan) versetzt. Man rührt das Gemisch 10 Minuten bei -78° und gibt dann innerhalb von 40 Minuten eine Lösung von 11,8 ml (0,1 Mol) 4-Methylbenzaldehyd in 100 ml Tetrahydrofuran zu. Das Reaktionsgemisch wird noch 20 Minuten bei -78° gerührt, dann auf Raumtemperatur gebracht und mit 100 ml gesättigter Ammoniumchlorid-Lösung versetzt. Die Wasserphase wird zweimal mit Aether extrahiert; die vereinigten organischen Phasen werden nacheinander mit gesättigter Kochsalzlösung und mit Wasser gewaschen, über Natriumsulfat getrocknet und eingeengt. Der Rückstand wird durch Flash-Chromatographie an 1000 g Silicagel (Elutionsmittel Methylenchlorid/Essigester 9:1) gereinigt. Man erhält 4-Hydroxy-4-(4-methylphenyl)-2-butinsäuremethylester als gelbes Oel.
MS: 204 ($M^+$), 189 (base peak) m/e

b) Eine Lösung von 8,1 g (39,7 mMol) 4-Hydroxy-4-(4-methylphenyl)-2-butinsäuremethylester in 100 ml Methylenchlorid wird bei 0° zu einer Suspension von 97 g (1,1 Mol) Mangandioxid in 200 ml Methylenchlorid getropft. Das Reaktionsgemisch wird 30 Minuten bei 0° gerührt, über Magnesiumsulfat filtriert und eingeengt. Kristallisation des Rückstandes aus Aether/Hexan liefert 3-(4-Methylbenzoyl)-propiolsäuremethylester vom Schmelzpunkt 55-56°.

Beispiel 26

a) Eine Lösung von 6,7 ml (80 mMol) Propiolsäuremethylester in 80 ml Tetrahydrofuran wird unter Argon bei -78° mit 53,1 ml n-Butyllithium (1,6M in Hexan) versetzt. Man rührt das Gemisch 10 Minuten bei -78° und gibt dann innerhalb von 30 Minuten eine Lösung von 11,94 g (80 mMol) 4-Dimethylaminobenzaldehyd in 80 ml Tetrahydrofuran zu. Das Reaktionsgemisch wird noch 20 Minuten bei -78° gerührt, dann auf Raumtemperatur gebracht und mit 120 ml gesättigter Ammoniumchlorid-Lösung versetzt. Die Wasserphase wird zweimal mit Aether extrahiert; die vereinigten organischen Phasen werden nacheinander mit gesättigter Kochsalzlösung und mit Wasser gewaschen, über Natriumsulfat getrocknet und eingeengt. Der Rückstand wird durch Flash-Chromatographie an 1000 g Silicagel (Elutionsmittel Methylenchlorid/Essigester 9:1) gereinigt. Man erhält 4-[4-(Dimethylamino)phenyl]-4-hydroxy-2-butinsäuremethylester als rotes Oel.
MS: 233 ($M^+$), 216 (base peak) m/e

b) Eine Lösung von 9,1 g (39 mMol) 4-[4-(Dimethylamino)phenyl]-4-hydroxy-2-butinsäuremethylester in 100 ml Methylenchlorid wird bei 0° zu einer Suspension von 96,7 g (1,1 Mol) Mangandioxid in 200 ml Methylenchlorid getropft. Das Reaktionsgemisch wird 1 Stunde bei 0° gerührt, über Magnesiumsulfat filtriert und eingeengt. Kristallisation des Rückstandes aus Methylenchlorid/Aether liefert 3-[4-(Dimethylamino)-benzoyl]propiolsäuremethylester vom Schmelzpunkt 112-114°.

Beispiel 27

a) Eine Lösung von 8,4 ml (0,1 Mol) Propiolsäuremethylester in 100 ml Tetrahydrofuran wird unter Argon bei -78° mit 69 ml n-Butyllithium (1,6M in Hexan) versetzt. Man rührt das Gemisch 10 Minuten bei -78° und gibt dann innerhalb von 40 Minuten eine Lösung von 13 ml (0,1 Mol) 4-(Methylthio)benzaldehyd in 100 ml Tetrahydrofuran zu. Das Reaktionsgemisch wird noch 20 Minuten bei -78° gerührt, dann auf Raumtemperatur gebracht und mit 150 ml gesättigter Ammoniumchlorid-Lösung versetzt. Die Wasserphase wird zweimal mit Aether extrahiert; die vereinigten organischen Phasen werden nacheinander mit gesättigter Kochsalzlösung und mit Wasser gewaschen, über Natriumsulfat getrocknet und eingeengt. Der Rückstand wird durch Flash-Chromatographie an 1000 g Silicagel (Elutionsmittel Methylenchlorid/Aether 4:1) gereinigt. Man erhält 4-Hydroxy-4-[4-(methylthio)phenyl]-2-butinsäuremethylester als rotes Oel.
MS: 236 ($M^+$) m/e

b) Eine Lösung von 11,6 g (49 mMol) 4-Hydroxy-4-[4-(methylthio)phenyl]-2-butinsäuremethylester in 300 ml Methylenchlorid wird bei 0° zu einer Suspension von 124 g (1,42 Mol) Mangandioxid in 300 ml Methylenchlorid getropft. Das Reaktionsgemisch wird 2 Stunden bei 0° gerührt, über Magnesiumsulfat filtriert und eingeengt. Kristallisation des Rückstandes aus Aether/Hexan liefert 3-[4-(Methylthio)benzoyl]-propiolsäuremethylester vom Schmelzpunkt 74-76°.

Beispiel 28

a) Eine Lösung von 12,5 ml (0,15 Mol) Propiolsäuremethylester in 150 ml Tetrahydrofuran wird unter Argon bei -78° mit 100 ml n-Butyllithium (1,6M in Hexan) versetzt. Man rührt das Gemisch 10 Minuten bei -78° und gibt dann innerhalb von 30 Minuten eine Lösung von 9,8 (80 mMol) 4-Hydroxybenzaldehyd in 150 ml Tetrahydrofuran zu. Das Reaktionsgemisch wird noch 20 Minuten bei -78° gerührt, dann auf Raumtemperatur gebracht und mit 200 ml gesättigter Ammoniumchlorid-Lösung versetzt. Die Wasserphase wird zweimal mit Aether extrahiert; die vereinigten organischen Phasen werden nacheinander mit gesättigter Natriumsulfat getrocknet und eingeengt. Der Rückstand wird durch Flash-Chromatographie an 1000 g Silicagel (Elutionsmittel Methylenchlorid/Essigester 9:1) gereinigt. Man erhält 4-Hydroxy-4-(4-hydroxyphenyl)-2-butinsäuremethylester als rotes Oel.
MS: 206 ($M^+$) m/e

b) Eine Lösung von 6,2 g (30 mMol) 4-Hydroxy-4-(4-hydroxyphenyl)-2-butinsäuremethylester in 200 ml Methylenchlorid und 50 ml Tetrahydrofuran wird bei 0° zu einer Suspension von 78,4 g (0,9 Mol) Mangandioxid in 200 ml Methylenchlorid getropft. Das Reaktionsgemisch wird 20 Stunden bei Raumtemperatur gerührt, über Magnesiumsulfat filtriert und eingeengt. Reinigung des Rückstandes durch Flash-Chromatographie an 600 g Silicagel (Elutionsmittel Methylenchlorid/Essigester 9:1) und anschliessende Kristallisation aus Aether/Hexan liefert 3-(4-Hydroxybenzoyl)propiolsäuremethyl ester vom Schmelzpunkt 84-86°.

Beispiel 29

a) Eine Lösung von 8,36 ml (0,1 Mol) Propiolsäuremethylester in 100 ml Tetrahydrofuran wird unter Argon bei -78° mit 68,8 ml n-Butyllithium (1,6M in Hexan) versetzt. Man rührt das Gemisch 10 Minuten bei -78° und gibt dann innerhalb von 40 Minuten eine Lösung von 16,6 g (0,1 Mol) 2,5-Dimethoxybenzaldehyd in 120 ml Tetrahydrofuran zu. Das Reaktionsgemisch wird noch 20 Minuten bei -78° gerührt, dann auf Raumtemperatur gebracht und mit 150 ml gesättigter Ammoniumchlorid-Lösung versetzt. Die Wasserphase wird zweimal mit Aether extrahiert; die vereinigten organischen Phasen werden nacheinander mit gesättigter Kochsalzlösung und mit Wasser gewaschen, über Natriumsulfat getrocknet und eingeengt. Der Rückstand wird durch Flash-Chromatographie an 1000 g Silicagel (Elutionsmittel Methylenchlorid/Essigester 9:1) gereinigt. Man erhält 4-(2,5-Dimethoxyphenyl)-4-hydroxy-2-butinsäuremethylester als gelbes Oel.
MS: 250 ($M^+$) m/e

b) Eine Lösung von 13,8 g (55 mMol) 4-(2,5-Dimethoxyphenyl)-4-hydroxy-2-butinsäuremethylester in 80 ml Methylenchlorid wird bei 0° zu einer Suspension von 139 g (1,6 Mol) Mangandioxid in 350 ml Methylenchlorid getropft. Das Reaktionsgemisch wird 1 Stunde bei 0° gerührt, über Magnesiumsulfat filtriert und eingeengt. Kristallisation des Rückstandes aus Methylenchlorid/Aether liefert 3-(2,5-Dimethoxybenzoyl)-propiolsäuremethylester vom Schmelzpunkt 70-71°.

Beispiel 30

Eine Lösung von 2,7 g (9,7 mMol) 3-(2,3,4-Trimethoxybenzoyl)propiolsäuremethylester in 30 ml Tetrahydrofuran wird bei 0° langsam mit 27 ml 3%iger Kaliumhydroxydlösung versetzt und anschliessend noch 1,5 Stunden bei 0° gerührt. Das Reaktionsgemisch wird mit Wasser versetzt und einmal mit Aether extrahiert. Die Aetherphase wird verworfen; die Wasserphase wird mit 1N Salzsäure auf pH 1 gestellt und zweimal mit Aether extrahiert. Die vereinigten organischen Phasen werden über Natriumsulfat getrocknet und eingeengt. Kristallisation des Rückstandes aus Essigester/Hexan liefert 3-(2,3,4-Trimethoxybenzoyl)-propiolsäure vom Schmelzpunkt 119-121°.

Beispiel 31

Eine Lösung von 2,8 g (10 mMol) 3-(3,4,5-Trimethoxybenzoyl)propiolsäuremethylester in 30 ml Tetrahydrofuran wird bei 0° langsam mit 28 ml 3%iger Kaliumhydroxydlösung versetzt und anschliessend noch 1,5 Stunden bei 0° gerührt. Das Reaktionsgemisch wird mit Wasser versetzt und einmal mit Aether extrahiert. Die Aetherphase wird verworfen; die Wasserphase wird mit 1N Salzsäure auf pH 1 gestellt und zweimal mit Aether extrahiert. Die vereinigten organischen Phasen werden über Natriumsulfat getrocknet und eingeengt. Kristallisation des Rückstandes aus Methylenchlorid/Aether/Hexan liefert 3-(3,4,5-Trimethoxybenzoyl)-propiolsäure vom Schmelzpunkt 130-132°.

Beispiel 32

Eine Lösung von 5,9 g (25,4 mMol) 3-[3,4-(Methylendioxy)benzoyl]propiolsäuremethylester in 60 ml Tetrahydrofuran wird bei 0° langsam mit 71 ml 3%iger Kaliumhydroxydlösung versetzt und noch 1 Stunde bei 0° gerührt. Das Reaktionsgemisch wird mit Wasser versetzt und einmal mit Aether extrahiert. Die Aetherphase wird verworfen; die Wasserphase wird mit 1N Salzsäure auf pH 1 gestellt und zweimal mit Aether extrahiert. Die vereinigten organischen Phasen werden über Natriumsulfat getrocknet und eingeengt. Kristallisation des Rückstandes aus Methylenchlorid/Aether/Hexan liefert 3-[3,4-(Methylendioxy)benzoyl]-propiolsäure vom Schmelzpunkt 122-124°.

Beispiel 33

Eine Lösung von 6,2 g (28 mMol) 3-(4-Methoxybenzoyl)-propiolsäuremethylester in 60 ml Tetrahydrofuran wird bei 0° langsam mit 80 ml 3%iger Kaliumhydroxydlösung versetzt und noch 1 Stunde bei 0° gerührt. Das Reaktionsgemisch wird mit Wasser versetzt und einmal mit Aether extrahiert. Die Aetherphase wird verworfen; die Wasserphase wird mit 1N Salzsäure auf pH 1 gestellt und zweimal mit Aether extrahiert.

Die vereinigten organischen Phasen werden über Natriumsulfat getrocknet und eingeengt. Ausfällen des Rückstandes aus Methylenchlorid/Aether/Hexan liefert 3-(4-Methoxybenzoyl)propiolsäure als amorphes Pulver.

MS: 204 ($M^+$) m/e

IR (KBr): 1714, 1641, 1588, 1510, 1262 $cm^{-1}$

Beispiel 34

Eine Lösung von 5 g (20 mMol) 3-(2,5-Dimethoxybenzoyl)-propiolsäuremethylester in 50 ml Tetrahydrofuran wird bei 0° langsam mit 56 ml 3%iger Kaliumhydroxydlösung versetzt und noch 1 Stunde bei 0° gerührt. Das Reaktionsgemisch wird mit Wasser versetzt und einmal mit Aether extrahiert. Die Aetherphase wird verworfen: die Wasserphase wird mit 1N Salzsäure auf pH 1 gestellt und zweimal mit Aeher extrahiert. Die vereinigten organischen Phasen werden über Natriumsulfat getrocknet und eingeengt. Kristallisation des Rückstandes aus Aether/Hexan liefert 3-(2,5-Dimethoxybenzoyl)propiolsäure vom Schmelzpunkt 91-93°.

Beispiel 35

a) Zu einer Lösung von 18,4 ml (0,3 Mol) Propiolsäure in 300 ml Aceton gibt man bei 0° 41,5 g (0,3 Mol) Kaliumcarbonat. Man rührt 4 Stunden bei Raumtemperatur, tropft dann 34,7 ml (0,3 Mol) 3,3-Dimethylallylbromid zu und kocht schliesslich noch während 20 Stunden am Rückfluss. Das abgekühlte Reaktionsgemisch wird am Rotationsverdampfer eingeengt; der Rückstand wird auf Eis/Wasser gegossen und zweimal mit Essigester extrahiert. Die vereinigten organischen Phasen werden über Natriumsulfat getrocknet und eingeengt. Destillation des Rückstandes im Wasserstrahlvakuum liefert Propiolsäure-(3-methyl-2-butenyl)ester vom Siedepunkt 77-78°/15 mmHg.

MS: 138 ($M^+$) m/e

b) Eine Lösung von 6,6 g ( 48 mMol) Propiolsäure-(3-methyl-2-butenyl)ester in 60 ml Tetrahydrofuran wird unter Argon bei -78° mit 30 ml n-Butyllithium (1,6M in Hexan) versetzt. Man rührt das Gemisch 10 Minuten bei -78° und gibt dann innerhalb von 30 Minuten eine Lösung von 9,4 g (48 mMol) 3,4,5-Trimethoxybenzaldehyd in 80 ml Tetrahydrofuran zu. Das Reaktionsgemisch wird noch 1 Stunde bei -78° gerührt, dann auf Raumtemperatur gebracht und mit 120 ml gesättigter Ammoniumchlorid-Lösung versetzt. Die Wasserphase wird zweimal mit Aether extrahiert; die organischen Phasen werden nacheinander mit gesättigter Kochsalzlösung und mit Wasser gewaschen, über Natriumsulfat getrocknet und eingeengt. Der Rückstand wird durch Flash-Chromatographie an 700 g Silicagel (Elutionsmittel Methylenchlorid/Essigester 9:1) gereinigt. Man erhält 4-Hydroxy-4-(3,4,5-trimethoxyphenyl)-2-butinsäure-(3-methyl-2-butenyl)ester als gelbes Oel.

MS: 334 ($M^+$) m/e

c) Eine Lösung von 10,7 g (32 mMol) 4-Hydroxy-4-(3,4,5-trimethoxyphenyl)-2-butinsäure-(3-methyl-2-butenyl)ester in 200 ml Methylenchlorid wird bei 0° zu einer Suspension von 84 g (0,97 Mol) Mangandioxid in 200 ml Methylenchlorid getropft. Das Reaktionsgemisch wird 2 Stunden bei 0° gerührt, über Magnesiumsulfat filtriert und eingeengt. Der Rückstand wird durch Flash-Chromtographie an 500 g Silicagel (Elutionsmittel Methylenchlorid/Essigester 9:1) gereinigt und aus Aether/Hexan kristallisiert. Man erhält 3-(3,4,5-Trimethoxybenzoyl)propiolsäure-(3-methyl -2-butenyl)ester von Schmelzpunkt 52-53°.

Beispiel 36

a) Eine Lösung von 10 g (72 mMol) Propiolsäure-(3-methyl-2-butenyl)ester in 100 ml Tetrahydrofuran wird unter Argon bei -78° mit 47 ml n-Butyllithium (1,6M in Hexan) versetzt. Man rührt das Gemisch 10 Minuten bei -78° und gibt dann innerhalb von 30 Minuten eine Lösung von 10,9 g (72 mMol) 3,4-Methylendioxybenzaldehyd in 120 ml Tetrahydrofuran zu. Das Reaktionsgemisch wird noch 30 Minuten bei -78° gerührt, dann auf Raumtemperatur gebracht und mit 120 ml gesättigter Ammoniumchlorid-Lösung versetzt. Die Wasserphase wird zweimal mit Aether extrahiert; die organischen Phasen werden nacheinander mit gesättigter Kochsalzlösung und mit Wasser gewaschen, über Natriumsulfat getrocknet und eingeengt. Der Rückstand wird durch Flash-Chromatographie an 1000 g Silicagel (Elutionsmittel Methylenchlorid/Essigester 9:1) gereinigt. Man erhält 4-Hydroxy-4-[3,4-(methylendioxy)phenyl]-2-butinsäure -(3-methyl-2-butenyl)ester als gelbes Oel.

MS: 288 ($M^+$) m/e

b) Eine Lösung von 17,2 g (60 mMol) 4-Hydroxy-4-[3,4-(methylendioxy)phenyl]-2-butinsäure - (3-methyl-2-butenyl)ester in 250 ml Methylenchlorid wird bei 0° zu einer Suspension von 156 g (1,8 Mol) Mangandioxid in 300 ml Methylenchlorid getropft. Das Reaktionsgemisch wird 1 Stunde bei 0° gerührt, über Magnesiumsulfat filtriert und eingeengt. Der Rückstand wird durch Flash-Chromatographie an 1000 g Silicagel (Elutionsmittel Methylenchlorid/Aether 4:1) gereinigt und aus Aether/Hexan kristallisiert. Man erhält 3-[3,4-(Methylendioxy)benzoyl]propiolsäure - (3-methyl-2-butenyl)ester vom Schmelzpunkt 52-53°.

Beispiel 37

a) Eine Lösung von 10 g (72 mMol) Propiolsäure-(3-methyl-2-butenyl)ester in 100 ml Tetrahydrofuran wird unter Argon bei -78° mit 47 ml n-Butyllithium (1,6M in Hexan) versetzt. Man rührt das Gemisch 10 Minuten bei -78° und gibt dann innerhalb von 30 Minuten eine Lösung von 8,8 ml (72 mMol) 4-Methoxybenzaldehyd in 100 ml Tetrahydrofuran zu. Das Reaktionsgemisch wird noch 30 Minuten bei -78° gerührt, dann auf Raumtemperatur gebracht und mit 100 ml gesättigter Ammoniumchlorid-Lösung versetzt. Die Wasserphase wird zweimal mit Aether extrahiert; die organischen Phasen werden nacheinander mit gesättigter Kochsalzlösung und mit Wasser gewaschen, über Natriumsulfat getrocknet und eingeengt. Der Rückstand wird durch Flash-Chromatographie an 1000 g Silicagel (Elutionsmittel Methylenchlorid/Essigester 9:1) gereinigt. Man erhält 4-Hydroxy-4-(4-methoxyphenyl)-2-butinsäure -(3-methyl-2-butenyl)ester als gelbes Oel.

MS: 274 ($M^+$) m/e

b) Eine Lösung von 10,7 g (39 mMol) 4-Hydroxy-4-(4-methoxyphenyl)-2-butinsäure -(3-methyl-2-butenyl)ester in 150 ml Methylenchlorid wird bei 0° zu einer Suspension von 101,7 g (1,17 Mol) Mangandioxid in 200 ml Methylenchlorid getropft. Das Reaktionsgemisch wird 1 Stunde bei 0° gerührt, über Magnesiumsulfat filtriert und eingeengt. Der Rückstand wird durch Flash-Chromatographie an 700 g Silicagel (Elutionsmittel Methylenchlorid/Aether 4:1) gereinigt. Man erhält 3-(4-Methoxybenzoyl)propiolsäure-(3-methyl-2-butenyl)ester als gelbes Oel.

MS: 272 ($M^+$) m/e

IR (Film): 1717, 1646, 1596, 1260, 1231, 1166 $cm^{-1}$

Beispiel 38

a) 12,3 ml (0,2 Mol) Propiolsäure werden zusammen mit 23,6 ml (0,2 Mol) Diäthylenglykolmonomethyläther, 1,5 g (8 mMol) p-Toluolsulfonsäuremonohydrat und 80 ml Toluol während 20 Stunden am Rückfluss gekocht, wobei das entstehende Reaktionswasser azeotrop abdestilliert und in einem Wasserabscheider gesammelt wird. Nach beendeter Wasserabscheidung wird das Reaktionsgemisch nacheinander mit gesättigter Natriumhydrogencarbonat-Lösung und mit Wasser gewaschen; die Toluolphase wird über Natriumsulfat getrocknet und eingeengt. Das Rohprodukt wird durch Flash-Chromatographie an 500 g Silicagel (Elutionsmittel Methylenchlorid/Essigester 9:1) gereinigt. Man erhält Propiolsäure-[2-(2-methoxyäthoxy)äthyl]ester als farbloses Oel.

b) Eine Lösung von 10 g (58 mMol) Propiolsäure-[2-(2-methoxyäthoxy)äthyl]ester in 80 ml Tetrahydrofuran wird unter Argon bei -78° mit 36 ml n-Butyllithium (1,6M in Hexan) versetzt. Man rührt das Gemisch 10 Minuten bei -78° und gibt dann innerhalb von 30 Minuten eine Lösung von 7 ml (58 mMol) 4-methoxybenzaldehyd in 60 ml Tetrahydrofuran zu. Das Reaktionsgemisch wird noch 30 Minuten bei -78° gerührt, dann auf Raumtemperatur gebracht und mit 150 ml gesättigter Ammoniumchlorid-Lösung versetzt. Die Wasserphase wird zweimal mit Aether extrahiert; die vereinigten organischen Phasen werden nacheinander mit gesättigter Kochsalzlösung und mit Wasser gewaschen, über Natriumsulfat getrocknet und eingeengt. Der Rückstand wird durch Flash-Chromatographie an 500 g Silicagel (Elutionsmittel Methylenchlorid/Aether 4:1) gereinigt. Man erhält 4-Hydroxy-4-(4-methoxyphenyl)-2-butinsäure -[2-(2-methoxyäthoxy)äthyl]ester als rotes Oel.

MS: 291 (M-OH) m/e

c) Eine Lösung von 9,8 g (32 mMol) 4-Hydroxy-4-(4-methoxyphenyl)-2-butinsäure -[2-(2-methoxyäthoxy)äthyl]ester in 100 ml Methylenchlorid wird bei 0° zu einer Suspension von 83 g (0,95 Mol) Mangandioxid in 150 ml Methylenchlorid getropft. Das Reaktionsgemisch wird 2 Stunden bei 0° gerührt, über Magnesiumsulfat filtriert und eingeengt. Der Rückstand wird durch Flash-Chromatographie an 800 g

Silicagel (Elutionsmittel Methylenchlorid/Aether 4:1) gereinigt. Man erhält 3-(4-methoxybenzoyl)propiolsäure -[2-(2-methoxyäthoxy)äthyl]ester als hellgelbes Oel.

MS: 248 ($M-CH_2=CH-OCH_3$) m/e

IR (Film): 1720, 1646, 1596, 1261 $cm^{-1}$

Beispiel 39

a) Eine Lösung von 10 g (58 mMol) Propiolsäure-[2-(2-methoxyäthoxy)äthyl]ester in 80 ml Tetrahydrofuran wird unter Argon bei -78° mit 36 ml n-Butyllithium (1,6M in Hexan) versetzt. Man rührt das Gemisch 10 Minuten bei -78° und gibt dann innerhalb von 30 Minuten eine Lösung von 11,4 g (8 mMol) 3,4,5-Trimethoxybenzaldehyd in 60 ml Tetrahydrofuran zu. Das Reakionsgemisch wird noch 30 Minuten bei -78° gerührt, dann auf Raumtemperatur gebracht und mit 150 ml gesättigter Ammoniumchlorid-Lösung versetzt. Die Wasserphase wird zweimal mit Aether extrahiert; die vereinigten organischen Phasen werden nacheinander mit gesättigter Kochsalzlösung und mit Wasser gewaschen, über Natriumsulfat getrocknet und eingeengt. Der Rückstand wird durch Flash-Chromatographie an 1000 g Silicagel (Elutionsmittel Methylenchlorid/Aether 4:1) gereinigt. Man erhält 4-Hydroxy-4-(3,4,5-trimethoxyphenyl)-2-butinsäure -[2-(2-methoxyäthoxy)äthyl]ester als rotes Oel.

MS: 368 ($M^+$) m/e

b) Eine Lösung von 8,5 g (23 mMol) 4-Hydroxy-4-(3,4,5-trimethoxphenyl)-2-butinsäure -[2-(2-methoxyäthoxy)äthyl]ester in 100 ml Methylenchlorid wird bei 0° zu einer Suspension von 60 g (0,69 Mol) Mangandioxid in 150 ml Methylenchlorid getropft. Das Rektionsgemisch wird 2 Stunden bei 0° gerührt, über Magnesiumsulfat filtriert und eingeengt. Der Rückstand wird durch Flash-Chromatographie an 700 g Silicagel (Elutionsmittel Methylenchlorid/Aether 4:1) gereinigt und anschliessend aus Aether/Hexan kristallisiert. Man erhält 3-(3,4,5-Trimethoxybenzoyl)propiolsäure -[2-(2-methoxyäthoxy)äthyl]ester vom Schmelzpunkt 33-34°.

Beispiel 40

a) Eine Lösung von 6,9 g (40 mMol) Propiolsäure-[2-(2-methoxyäthoxy)äthyl]ester in 60 ml Tetrahydrofuran wird unter Argon bei -78° mit 25 ml n-Butyllithium (1,6M in Hexan) versetzt. Man rührt das Gemisch 10 Minuten bei -78° und gibt dann innerhalb von 30 Minuten eine Lösung von 6 g (40 mMol) 3,4-Methylendioxybenzaldehyd in 60 ml Tetrahydrofuran zu. Das Reaktionsgemisch wird noch 30 Minuten bei -78° gerührt, dann auf Raumtemperatur gebracht und mit 100 ml gesättiger Ammoniumchlorid-Lösung versetzt. Die Wasserphase wird zweimal mit Aether extrahiert; die vereinigten organischen Phasen werden nacheinander mit gesättigter Kochsalzlösung und mit Wasser gewaschen, über Natriumsulfat getrocknet und eingeengt. Der Rückstand wird durch Flash-Chromatographie an 800 g Silicagel (Elutionsmittel Methylenchlorid/Aether 4:1) gereinigt. Man erhält 4-Hydroxy-4-[3,4-(methylendioxy)phenyl]-2-butinsäure -[2-(2-methoxyäthoxy)äthyl]ester als gelbes Oel.

MS: 322 ($M^+$) m/e

b) Eine Lösung von 7,9 g (24,5 mMol) 4-Hydroxy-4-[3,4-(methylendioxy)phenyl]-2-butinsäure -[2-(2-methoxyäthoxy)äthyl]ester in 100 ml Methylenchlorid wird bei 0° zu einer Suspension von 64 g (0,74 Mol) Mangandioxid in 150 ml Methylenchlorid getropft. Das Reaktionsgemisch wird 2 Stunden bei 0° gerührt, über Magnesiumsulfat filtriert und eingeengt. Der Rückstand wird durch Flash-Chromatographie an 800 g Silicagel (Elutionsmittel Methylenchlorid/Aether 4:1) gereinigt. Man erhält 3-[3,4-(Methylendioxy)benzoyl]-propiolsäure -[2-(2-methoxyäthoxy)äthyl]ester als gelbes Oel.

MS: 320 ($M^+$) m/e

IR (Film): 1719, 1643, 1601, 1445, 1261 $cm^{-1}$

Beispiel 41

Zu einer Lösung von 2,0 g (7,99 mMol) 4-Hydroxy-1-(3,4,5-trimethoxypheny)-2-butin-1-on in 20 ml Methylenchlorid werden bei 0° gleichzeitig 0,815 g Acetylchlorid und 0,827 g Pyridin zugegeben. Das Reaktionsgemisch wird 1 Stunde bei 0° gerührt, worauf man mit 30 ml Phosphat-Puffer (pH 6) versetzt und die Wasserphase zweimal mit Methylenchlorid extrahiert. Die vereinigten organischen Phasen werden über Magnesiumsulfat getrocknet und eingedampft. Der Rückstand wird aus Essigester Hexan umkristallisiert, und man erhält 3-(3,4,5-Trimethoxybenzoyl)-2-propinyl-acetat vom Schmelzpunkt 104-105°.

Beispiel 42

Zu einer Lösung von 2,0 g (7,99 mMol) 4-Hydroxy-1-(3,4,5-trimethoxypheny)-2-butin-1-on in 25 ml Methylenchlorid werden bei 0° gleichzeitig 2,21 g (9,59 mMol) 3,4,5-Trimethoxybenzoylchlorid in 10 ml Methylenchlorid und 1 ml Pyridin zugegeben. Das Reaktionsgemisch wird 30 Minuten bei 0° und 1,5 Stunden bei Raumtemperatur gerührt, worauf man 30 ml 0,5M Salzsäure und 100 ml Methylenchlorid zugibt und die wässrige Phase zweimal mit Methylenchlorid extrahiert. Die vereinigten organischen Phasen werden über Magnesiumsulfat getrocknet und eingedampft, und der Rückstand wird an Kieselgel mit Aether chromatographiert. Nach Umkristallisation aus Essigester/Hexan erhält man 3-(3,4,5-Trimethoxybenzoyl)-2-propinyl-3,4,5-trimethoxybenzoat vom Schmelzpunkt 106-108°.

Beispiel 43

Zu einer Lösung von 10 ml (69,8 mMol) 3,3-Diäthoxy-1-propin in 210 ml absolutem Tetrahydrofuran werden 43,6 ml n-Butyllithium-Lösung (1,6M in Hexan) bei -78° zugegeben. Das Gemisch wird 30 Minuten bei -78° gerührt und bei -40° mit 13,70 g (69,8 mMol) 3,4,5-Trimethoxybenzaldehyd in 60 ml Tetrahydrofuran versetzt. Das Reaktionsgemisch wird 1 Stunde bei 0° gerührt und mit 100 ml gesättigter Ammoniumchlorid-Lösung und 150 ml Aether versetzt. Die Wasserphase wird zweimal mit Aether extrahiert; die vereinigten organischen Phasen werden über Magnesiumsulfat getrocknet und eingedampft. Der Rückstand wird in 100 ml Methylenchlorid gelöst und bei 0° zu einer Suspension von 176,8 g Mangandioxid in 300 ml Methylenchlorid getropft. Das Reaktionsgemisch wird 20 Minuten bei 0° gerührt, über Magnesiumsulfat filtriert und eingedampft. Der Rückstand wird aus iso-Octan bei 0° kristallisiert, und man erhält 4,4-Diäthoxy-1-(3,4,5-trimethoxyphenyl)-2-butin-1-on vom Schmelzpunkt 52-54°.

Beispiel 44

Zu einer Lösung von 2,0 g (6,2 mMol) 4,4-Diäthoxy-1-(3,4,5-trimethoxyphenyl)-2-butin-1-on in 10 ml Dioxan werden bei Raumtemperatur 3 ml 60%ige Perchlorsäure gegeben. Das Reaktionsgemisch wird 2 Stunden bei Raumtemperatur gerührt und mit 20 ml Aether und 10 ml Wasser versetzt. Die Wasserphase wird dreimal mit Aether extrahiert; die vereinigten Aetherphasen werden über Magnesiumsulfat getrocknet und eingedampft. Chromatographie des Rückstandes an Kieselgel liefert neben unumgesetztem Ausgangsmaterial 3-(3,4,5-Trimethoxybenzoyl)-2-propinal vom Schmelzpunkt 86° (Zers.).
MS: 248 ($M^+$) m/e

Beispiel 45

Zu einer Lösung von 2,0 g (7,83 mMol) N,3,4,5-Tetramethoxy-N-methylbenzoylamid in 20 ml absolutem Tetrahydrofuran tropft man bei -40° eine Lösung, hergestellt bei -40° aus 0,66 g (9,40 mMol) 3-Butin-2-ol, 12,55 ml n-Butyllithium-Lösung (1,6M in Hexan) und 6 ml Hexamethylphosphortriamid in 20 ml absolutem Tetrahydrofuran. Das Reaktionsgemisch wird 15 Minuten bei -40° gerührt, langsam auf 0° erwärmt und je 1 Stunde bei 0° und bei Raumtemperatur gerührt, worauf man 20 ml 5%ige äthanolische Salzsäure und 100 ml Aether zugibt. Die organische Phase wird dreimal mit Wasser gewaschen, über Magnesiumsulfat getrocknet und eingedampft. Nach Chromatographie des Rückstands an Kieselgel mit Aether und Kristallisation aus Essigester/Hexan erhält man 4-Hydroxy-1-(3,4,5-trimethoxyphenyl)-2-pentin-1-on vom Schmelzpunkt 77-79°.

Beispiel 46

a) Zu einer Lösung von 11,72 ml (0.115 Mol) Propiolsäureäthylester in 195 ml Tetrahydrofuran/Aether/Hexan (4:2:1) werden bei -110° 72 ml n-Butyllithium-Lösung (1,6M in Hexan) zugegeben. Das Gemisch wird 20 Minuten bei -110° gerührt und dann mit 15,0 g (76,4 mMol) 3,4,5-Trimethoxybenzaldehyd in 30 ml Tetrahydrofuran versetzt. Das Reaktionsgemisch wird danach 1,5 Stunden bei -78° gerührt und bei -30° mit 100 ml gesättigter Ammoniumchlorid-Lösung und 100 ml Essigester versetzt. Die Wasserphase wird zweimal mit Essigester extrahiert; die vereinigten organischen Phasen werden über Magnesiumsulfat getrocknet und eingedampft. Der Rückstand wird an 500 g Kieselgel mit Essigester/Hexan (1:1) chromatographiert, wonach 4-Hydroxy-4-(3,4,5-trimethoxyphenyl)-2-butinsäureäthylester erhalten wird.

IR (Film): 2220(w), 1712(s), 1595(s), 1244(s), 1127(s).

b) Eine Lösung von 21,6 g (73,4 mMol) von 4-Hydroxy-4-(3,4,5-trimethoxyphenyl)-2-butinsäureäthylester in 80 ml Methylenchlorid wird bei 0° zu einer Suspension von 191,4 g (2,2 Mol) Mangandioxid in 330 ml Methylenchlorid getropft. Das Reaktionsgemisch wird 30 Minuten bei 0° gerührt, über Magnesiumsulfat filtriert und eingedampft. Der Rückstand wird aus Essigester/Hexan über Nacht bei 0° kristallisiert. Man erhält 3-(3,4,5-Trimethoxybenzoyl)-2-propiolsäureäthylester vom Schmelzpunkt 61-62,5°.

Beispiel 47

Zu einer Lösung von 3,0 g (13,5 mMol) 1-(3,4,5-Trimethoxyphenyl)-2-propin-1-ol in 40 ml Aether werden bei -78° 8,7 ml n-Butyllithium-Lösung (1,6M in Hexan) zugegeben. Das Rektionsgemisch wird auf -30° gebracht, worauf man bei -30° 1,8 ml (14,2 mMol) frisch destilliertes Trimethylsilylchlorid zugibt. Danach wird auf 0° erwärmt, 1 Stunde gerührt und auf -78° gekühlt, worauf 7,8 ml n-Butyllithium-Lösung (1,6M in Hexan) zugegeben werden. Das Reaktionsgemisch wird 1 Stunde bei -30° gerührt und dann bei -30° mit 2,77 ml (20,25 mMol) Chlorameisensäure-butylester versetzt, worauf man 30 Minuten bei -30° und 1 Stunde bei 0° rührt und je 150 ml 1N Salzsäure und Essigester zugibt. Anschliessend gibt man 30 ml 25%ige Salzsäure zu, schüttelt das Gemisch während 10 Minuten heftig im Scheidetrichter, extrahiert die Wasserphase zweimal mit Essigester, trocknet die vereinigten organischen Phasen über Magnesiumsulfat und destilliert das Lösungsmittel ab. Das als Rückstand verbleibende Rohprodukt wird in 10 ml Methylenchlorid aufgenommen und bei 0° zu einer Suspension von 35,2 g (0,405 Mol) Mangandioxid in 50 ml Methylenchlorid getropft. Das Reaktionsgemisch wird 1,5 Stunden bei 0° gerührt, über Magnesiumsulfat filtriert und eingedampft. Nach Chromatographie des Rückstands an Kieselgel mit Essigester/Hexan (1:4) erhält man 3-(3,4,5-Trimethoxybenzoyl)propiolsäurebutylester als leicht gelbliches Oel.

IR (Film) 1719(s), 1647(m), 1582(m), 1501(m), 1415(m), 1334(s), 1248(s), 1128(s)

Beispiel 48

a) Zu einer Lösung von 12,0 g (53,98 mMol) α-Aethinyl-3,4,5-trimethoxybenzylalkohol in 100 ml Methylenchlorid und 9,65 ml 1,8-Diazabicyclo[5.4.0]undec-7-en wird bei 0° eine Lösung von 9,76 g (64,78 mMol) tert.-Butyldimethylsilylchlorid in 50 ml Methylenchlorid zugetropft. Das Reaktionsgemisch wird 30 Minuten bei 0° und 1 Stunde bei Raumtemperatur gerührt, worauf man 100 ml Phosphatpuffer (pH 6) zugibt. Die wässrige Phase wird zweimal mit 50 ml Aether extrahiert; die vereinigten organischen Phasen werden über Magnesiumsulfat getrocknet und eingedampft. Der Rückstand wird an 500 g Kieselgel mit Aether/Petroläther (4:1) chromatographiert, wonach 1-[(tert)Butyldimethylsiloxy]-1-(3,4,5-trimethoxyphenyl) -2-propin erhalten wird.

IR (Film): 3282(w), 2955(s), 2932(s), 1594(s), 1506(m), 1463(s), 1417(m), 1332(m), 1097(s), 837(s).

b) Zu einer Lösung von 3,0 g (8,9 mMol) 1-[(tert)Butyldimethylsiloxy] -1-(3,4,5-trimethoxyphenyl)-2-propin in 25 ml absolutem Tetrahydrofuran werden bei -78° 5,6 ml n-Butyllithium-Lösung (1,6M in Hexan) zugegeben. Das Reaktionsgemisch wird langsam auf 0° gebracht und noch 15 Minuten bei 0° gerührt, worauf man bei -78° 1,1 ml N,N-Dimethylcarbamoylchlorid zugibt. Das Reaktionsgemisch wird 30 Minuten bei -78° und 2 Stunden bei 0° gerührt, mit je 10 ml 2N Salzsäure und Dioxan versetzt und 12 Stunden bei Raumtemperatur gerührt. Nach Zugabe von je 50 ml Aether und Wasser wird die organische Phase über Magnesiumsulfat getrocknet und eingedampft. Der Rückstand wird an Kieselgel mit Essigester chromatographiert, wonach ein leicht gelbes viskoses Oel erhalten wird, welches in 5 ml Methylenchlorid aufgenommen und bei 0° zu einer Suspension von 10,2 g Mangandioxid in 15 ml Methylenchlorid zugegeben wird. Das Reak tionsgemisch wird 2,5 Stunden bei 0° gerührt, über Magnesiumsulfat filtriert und eingedampft. Der Rückstand wird aus Essigester/Hexan bei 0° kristallisiert, wobei man N.N-Dimethyl-3-(3,4,5-trimethoxybenzoyl)propiolamid vom Schmelzpunkt 118-119° erhält.

Beispiel 49

a) Eine Lösung von 11,7 g (50 mMol) 3-Trimethylsilylorthopropiolsäure-triäthylester (vgl. G. Boche & J. Bigalke, Tetrahedron Letters 1984, 955) in 100 ml trockenem Tetrahydrofuran wird bei -5° bis 0° tropfenweise mit 31,25 ml 1.6M Butyllithiumlösung in Hexan versetzt. Nach beendeter Zugabe wird 1 Stunde bei -10° gerührt, dann mit weiteren 3,0 ml 1.6M Butyllithiumlösung in Hexan versetzt und noch 10 Minuten bei 0° gerührt. Hierauf wird bei -5° eine Lösung von 9,81 g (50 mMol) 3,4,5-Trimethoxybenzaldehyd in 60 ml Tetrahydrofuran zugetropft; die Reaktionsmischung wird 10 Minuten bei -10° gehalten und anschliessend innerhalb einer Stunde auf Raumtemperatur erwärmen gelassen. Zur Aufarbeitung versetzt man mit gesättigter $NaHCO_3$-Lösung, verdünnt mit Aether/Hexan 1:1, trennt die Phasen und extrahiert die wässrige Phase mit Hexan/Aether. Die vereinigten organischen Phasen werden mit Wasser, gesättigter Natriumbicarbonat-und Kochsalz-Lösung gewaschen, über Natriumsulfat getrocknet, filtriert und eingedampft. Chromatographie an Kieselgel mit Hexan/Essigsäureäthylester 30%→50%/Triäthylamin 0,1% liefert 4-Hydroxy-4-(3,4,5-trimethoxyphenyl)-2-butinorthosäure-triäthylester als gelbes Oel.
IR Film): 3465 (br, OH); 2230 (C≡C).
NMR (250 MHz, $CDCl_3$): 6,79 (s, 2 aromat H); 5,48 (d, J = 6,5, H-C(4)); 3,87 (s, 2 $OCH_3$); 3,85 (s, 1 $OCH_3$); 3,71 (q, J = 7, 3 $O\underline{CH_2}CH_3$); 2,37 (d, J = 6,5, OH); 1,24 (t, J = 7, 3 $OCH_2\underline{CH_3}$).
MS: 368 (44, $M^+$); 323 (88, $M^+$-OEt); 197 (63): 169 (100).

b) Eine Lösung von 8,49 g (23 mMol) 4-Hydroxy-4-(3,4,5-trimethoxyphenyl) -2-butinorthosäure-triäthylester in 100 ml Dichlormethan wird mit 10,0 g Mangandioxid versetzt worauf die Suspension während 3,5 Stunden bei Raumtemperatur gerührt wird. Danach filtriert man über ein Bett von Hyflo, dampft das Filtrat ein und chromatographiert den Rückstand an Kieselgel mit Hexan/Essigsäureäthylester 12%→20%/Triäthylamin 0,1%. Dabei wird 4-Oxo-4-(3,4,5-trimethoxyphenyl) -2-butinorthosäuretriäthylester als gelbliches, viskoses Oel erhalten, welches beim Aufbewahren bei -15° erstarrt. Eine Probe zur Analyse wird durch Kristallisation aus Pentan bei -15° gewonnen; Smp. 29-31°.
IR Film): 2220 (C≡C); 1647 (C=O konj.).
NMR (250 MHz, $CDCl_3$): 7,42 (s, 2 aromt. H); 3,95 (s, 1 $OCH_3$); 3,92 (s, 2 $OCH_3$); 3,79 (q, J=7, 3 $O\underline{CH_2}CH_3$); 1,28 (t, J=7, 3 $OCH_2\underline{CH_3}$).
MS: 366 (44, $M^+$); 321 (100, $M^+$-OEt); 195 (64); 147 (42, $(EtO)_3C^+$).
Anal. ber. für $C_{19}H_{26}O_7$ (366,41):
C 62,28, H 7,15;
gef.
C 61,96, H 7,29.

Beispiel 50

a) Eine Lösung von 29,6 ml (0,5 Mol) 2-Propin-1-ol in 500 ml Tetrahydrofuran wird bei -78° unter Argon mit 67,3 g (0,6 Mol) Kalium-tert.-butylat versetzt. Man rührt 4 Stunden bei -78°, gibt dann 69,4 ml (0,6 Mol) 3,3-Dimethylallylbromid zu und rührt nochmals 3 Stunden bei -78° und 20 Stunden bei Raumtemperatur. Das Reaktionsgemisch wird auf Eis/Wasser gegossen und zweimal mit Aether extrahiert. Die vereinigten organischen Phasen werden über Magnesiumsulfat getrocknet und eingeengt. Man erhält 1-[(3-Methyl-2-butenyl)oxy] -2-propin als gelbes Oel, welches ohne Reinigung weiterverwendet wird.
MS: 123 (M-H), 109 (M-$CH_3$) m/e.

b) Eine Lösung von 15 g (121 mMol) 1-[(3-Methyl-2-butenyl)oxy] -2-propin in 150 ml Tetrahydrofuran wird unter Argon bei -78° mit 83 ml n-Butyllithium (1,6M in Hexan) versetzt. Man lässt das Gemisch 30 Minuten bei -40° rühren, kühlt wieder auf -78° ab und gibt dann innerhalb von 40 Minuten eine Lösung von 26,1 g (133 mMol) 3,4,5-Trimethoxybenzaldehyd in 150 ml Tetrahydrofuran zu. Das Reaktionsgemisch wird noch 1 Stunde bei -78° gerührt, dann auf Raumtemperatur erwärmt und anschliessend mit 150 ml gesättigter Ammoniumchlorid-Lösung versetzt. Die Wasserphase wird zweimal mit Aether extrahiert. Die vereinigten organischen Phasen werden nacheinander mit gesättigter Kochsalzlösung und mit Wasser gewaschen, über Magnesiumsulfat getrocknet und eingeengt. Der Rückstand wird durch Flash-Chromatographie an 1000 g Silicagel (Elutionsmittel Methylenchlorid/Aether 4:1) gereinigt. Man erhält 1-Hydroxy-4-[-(3-methyl-2-butenyl)oxy] -1-(3,4,5-trimethoxyphenyl)-2-butin als gelbes Oel.
MS: 320 ($M^+$), 197 (base peak) m/e.
IR (Film): 3124, 1594, 1505, 1324, 1127 $cm^{-1}$.

c) Eine Lösung von 30 g (93.6 mMol) 1-Hydroxy-4-[(3-methyl-2-butenyl)oxy]-1-(3,4,5-trimethoxyphenyl) -2-butin in 350 ml Methylenchlorid wird bei 0° zu einer Suspension von 122 g (1,4 Mol) Mangandioxid in 500 ml Methylenchlorid getropft. Das Reaktionsgemisch wird 3 Stunden bei 0° gerührt, über Magnesiumsulfat filtriert und eingeengt. Flash-Chromatographie des Rückstandes an 1000 g Silicagel (Elutionsmittel Methylenchlorid/Essigester 9:1) und anschliessende Kristallisation des Produktes aus Aether Hexan liefert 4-[(3-Methyl-2-butenyl)oxy]-1-(3,4,5-trimethoxyphenyl)-2-butin-1-on vom Schmelzpunkt 32°.

Beispiel 51

a) Eine Lösung von 20 g (161 mMol) 1-[(3-Methyl-2-butenyl)oxy] -2-propin in 200 ml Tetrahydrofuran wird unter Argon bei -78° mit 111 ml n-Butyllithium (1,6M in Hexan) versetzt. Man lässt das Gemisch 30 Minuten bei -40° rühren, kühlt wieder auf -78° ab und gibt dann innerhalb von 40 Minuten eine Lösung von 26,6 g (177 mMol) 3,4-Methylendioxybenzaldehyd in 150 ml Tetrahydrofuran zu. Das Reaktionsgemisch wird noch 1 Stunde bei -78° gerührt, dann auf Raumtemperatur erwärmt und anschliessend mit 150 ml gesättigter Ammoniumchlorid-Lösung versetzt. Die Wasserphase wird zweimal mit Aether extrahiert. Die vereinigten organischen Phasen werden nacheinander mit gesättigter Kochsalzlösung und mit Wasser gewaschen, über Magnesiumsulfat getrocknet und eingeengt. Der Rückstand wird durch Flash-Chromatographie an 1000 g Silicagel (Elutionsmittel Methylenchlorid/Essigester 9:1) gereinigt. Man erhält 1-Hydroxy-4-[(3-methyl-2-butenyl)oxy]-1-[3,4-(methylendioxy)phenyl]-2-butin als gelbes Oel.
MS: 274 ($M^+$), 243, 149, 131 m/e.
IR (Film): 3393, 1486, 1443, 1246, 1039 $cm^{-1}$.

b) Eine Lösung von 34,2 g (125 mMol) 1-Hydroxy-4-[(3-methyl-2-butenyl)oxy]-1-[3,4(methylendioxy)-phenyl] -2-butin in 350 ml Methylenchlorid wird bei 0° zu einer Suspension von 163 g (1,87 Mol) Mangandioxid in 500 ml Methylenchlorid getropft. Das Reaktionsgemisch wird 2 Stunden bei 0° gerührt, über Magnesiumsulfat filtriert und eingeengt. Das Rohprodukt wird bei -50° aus Aether/Hexan umkristallisiert. Man erhält 4-[(3-Methyl-2-butenyl)oxy] -1-[3,4-(methylendioxy)phenyl] -2-butin-1-on als hellgelbe Kristalle, die bei Raumtemperatur schmelzen.
MS: 272 ($M^+$, 149 (base peak) m/e.
IR (Film): 2230, 1640, 1602, 1444, 1262 $cm^{-1}$.

Beispiel 52

a) Zu einer Lösung von 26,4 ml (0,43 Mol) Propiolsäure in 400 ml Methylenchorid gibt man bei 25°, wenn nötig unter Eiskühlung, 64 ml (0,43 Mol) 1,8-Diazabicyclo[5.4.0]undec-7-en. Man rührt 2 Stunden bei 25°, tropft dann 56 ml (415 mMol) 4-Methoxybenzylchlorid zu und rührt schliesslich noch während 2 Tagen bei 25°. Das Reaktionsgemisch wird nacheinander mit 200 ml gesättigter Natriumhydrogencarbonat-Lösung und zweimal je 200 ml Wasser gewaschen. Die organische Phase wird über Natriumsulfat getrocknet und eingeengt. Der Rückstand wird durch Flash-Chromatographie an 1000 g Silicagel (Elutionsmittel Hexan/Essigester 9:1) gereinigt. Man erhält Propiolsäure-(4-methoxybenzyl)ester als farbloses Oel.
MS: 190 ($M^+$), 121 (base peak) m/e.

b) Eine Lösung von 46,7 g (245,5 mMol) Propiolsäure-(4-methoxybenzyl)ester in 600 ml Tetrahydrofuran wird unter Argon bei -78° innerhalb von 1,5 Stunden mit 169 ml n-Butyllithium (1,6M in Hexan) versetzt. Man rührt das Gemisch 10 Minuten und gibt dann innerhalb von 1 Stunde eine Lösung von 36,8 g (245,5 mMol) 3,4-Methylendioxybenzaldehyd in 200 ml Tetrahydrofuran zu. Das Reaktionsgemisch wird noch 15 Minuten bei -78° gerührt und dann auf ein Gemisch von 300 g Eis und 300 ml gesättigter Ammoniumchlorid-Lösung gegossen. Die Wasserphase wird zweimal mit Aether extrahiert; die organischen Phasen werden nacheinander mit gesättigter Kochsalzlösung und mit Wasser gewaschen, über Natriumsulfat getrocknet und eingeengt. Der Rückstand wird durch Flash-Chromatographie an 3,5 kg Silicagel (Elutionsmittel Methylenchlorid/Essigester 9:1) gereinigt. Man erhält 4-Hydroxy-4-(3,4-methylendioxyphenyl) -2-butinsäure-(4-methoxybenzyl)ester als braunes Oel.
MS: 340 ($M^+$), 121 (base peak) m/e.
IR (Film): 3400, 2230, 1710, 1612, 1513, 1249 $cm^{-1}$.

c) Eine Lösung von 53 g (155 mMol) 4-Hydroxy-4-(3,4-methylendioxyphenyl)-2-butinsäure-(4-methoxybenzyl)ester in 400 ml Methylenchlorid wird bei 0° zu einer Suspension von 338 g (3,9 Mol) Mangandioxid in 800 ml Methylenchlorid getropft. Das Reaktionsgemisch wird 1 Stunde bei 0° gerührt, mit 4 Spatelspitzen Natriumsulfat versetzt, über Dicalit abgenutscht und eingeengt. Kristallisation des Rückstandes aus Aether liefert 3-[3,4-(Methylendioxy)benzoyl]propiolsäure -(4-methoxybenzyl)ester vom Schmelzpunkt 113-114°.

Beispiel 53

Eine Lösung von 36,3 g (107 mMol) 3-[3,4-(Methylendioxy)benzoyl]propiolsäure -(4-methoxybenzyl)-ester in 300 ml Methylenchlorid wird bei Raumtemperatur mit 16,4 ml (215 mMol) wasserfreier Trifluoressigsäure versetzt. Das Reaktionsgemisch wird bis zur beginnenden Kristallisation bei Raumtemperatur gerührt, dann auf 0° abgekühlt und noch 30 Minuten weitergerührt. Die ausgefallenen Kristalle werden abgenutscht, mit kaltem Methylenchlorid gewaschen und im Hochvakuum getrocknet. Man erhält 3-[3,4-(Methylendioxy)benzoyl]propiolsäure vom Schmelzpunkt 137°.

Beispiel A

Kristalline Verbindungen der Formeln I und II und pharmazeutisch verwendbare Salze von Verbindungen der Formel I können als Wirkstoffe zur Herstellung von Hartgelatinekapseln verwendet werden, deren Inhalt pro Kapsel folgende Zusammensetzung aufweist:

| | |
|---|---|
| Wirkstoff | 50-250,0 mg |
| Milchzucker pulv. | 40,0 mg |
| Milchzucker krist. | 230- 30,0 mg |
| Maisstärke weiss | 20,0 mg |
| Talk | 8,0 mg |
| Magnesiumstearat | 2,0 mg |
| Füllgewicht pro Kapsel | 250,0 mg |

Der Wirkstoff und die Hilfsstoffe werden miteinander vermischt, und das Gemisch wird in Hartgelatinekapseln geeigneter Grösse abgefüllt. Erforderlichenfalls werden die Kapseln anschliessend mit einem magensaftresistenten Lack, bestehend aus Hydroxypropylmethylcellulosephthalat, versehen.

Beispiel B

Nicht kristalline Verbindungen der Formeln I und II können wie nachstehend beschrieben als Wirkstoffe zur Herstellung von Weichgelatinekapseln verwendet werden; die verwendeten Abkürzungen haben dabei folgende Bedeutung:

BHA = Butyliertes Hydroxyanisol
BHT = Butyliertes Hydroxytoluol
PEG = Polyäthylenglykol

a) 0,2 mg BHA und 1,0 mg Ascorbylpalmitat werden in 400 mg PEG 400 bei Raumtemperatur unter Stickstoffatmosphäre gelöst. Die Lösung wird mit 50-250 mg Wirkstoff bei Raumtemperatur unter Stickstoff versetzt. Nachdem alles gelöst ist, wird das erhaltene Gemisch in flüssiger Form in Weichgelatinekapseln abgefüllt.

b) 300 mg PEG 400 und 100 mg PEG 4000 werden unter Stickstoff erwärmt, bis sich das Gemisch verflüssigt hat. Danach werden unter Stickstoff 0,1 mg BHA, 0,1 mg BHT und 1,0 mg Ascorbylpalmitat zugesetzt. Nachdem sich alles gelöst hat, werden 50-250 mg Wirkstoff unter Stickstoff zugefügt und unter Durchmischung gelöst. Die Flüssigkeit wird dann in Weichgelatinekapseln abgefüllt.

c) 0,2 mg BHA, 0,2 mg BHT und 1,0 mg Ascorbylpalmitat werden in 400 mg Polysorbat-80 bei Raumtemperatur unter Stickstoff gelöst. Das Gemisch wird unter Stickstoff mit 50-250 mg Wirkstoff versetzt. Nachdem sich alles gelöst hat, wird die Flüssigkeit in Weichgelatinekapseln abgefüllt.

d) Ein Gemisch von je 200 mg Polysorbat-60 und Polysorbat-80 wird erwärmt. Das erhaltene flüssige Gemisch wird unter Stickstoff mit 0,2 mg BHA, 1,0 mg α-Tocopherol und 2,0 mg Ascorbylpalmitat versetzt. Nachdem alles gelöst ist, werden 50-250 mg Wirkstoff unter Stickstoff zugesetzt. Nach Durchmischung bis zur vollständigen Lösung wird das erhaltene Gemisch in Weichgelatinekapseln abgefüllt.

## Ansprüche

1. Propiolophenonderivate der allgemeinen Formel

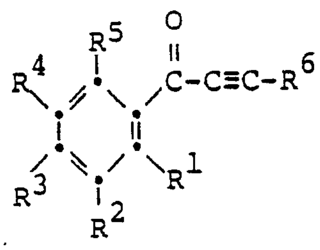

I

worin

$R^1$, $R^2$, $R^3$, $R^4$ und $R^5$ je Wasserstoff, Halogen, niederes Alkyl, niederes Alkoxy-niederes-alkyl, Hydroxy, niederes Alkoxy, niederes Alkenyloxy, niederes Alkinyloxy, niederes Alkoxy-niederes-alkoxy, Acyloxy, Aryl-niederes-alkoxy, niederes Alkylthio, niederes Alkoxy-niederes-alkylthio, niederes Alkenylthio, niederes Alkinylthio, Aryl-niederes-alkylthio, gegebenenfalls substituiertes Amino oder Trifluormethyl oder zwei benachbarte dieser Substituenten gemeinsam und zusammen mit den Kohlenstoffatomen, an welche sie gebunden sind, einen 5-bis 7-gliedrigen Ring bedeuten, wobei von den Substituenten $R^1$ bis $R^5$ mindestens zwei Wasserstoff bedeuten und mindestens einer von Wasserstoff verschieden ist; und

$R^6$ Wasserstoff, niederes Alkyl oder einen Rest der Formel

$-COOR^7$, (a)  $-CONR^8R^9$, (b)  $-C(R^{10})=O$, (c)

$-C(R^{11})(OR^{12})_2$, (d)  $-C(OR^{13})_3$ (e) oder

$-C(R^{14})(R^{15})OR^{16}$ (f) bedeutet;

$R^7$ Wasserstoff, niederes Alkyl, niederes Alkenyl, niederes Alkoxy-niederes-alkyl, niederes Alkoxy-niederes-alkoxy-niederes-alkyl, Aryl oder Aryl-niederes-alkyl bedeutet;

$R^8$ und $R^9$ je Wasserstoff oder niederes Alkyl oder gemeinsam und zusammen mit dem Stickstoffatom einen 5-bis 7-gliedrigen gesättigten heterocyclischen Rest bedeuten;

$R^{10}$ Wasserstoff, niederes Alkyl, Aryl oder Aryl-niederes-alkyl bedeutet;

$R^{11}$ Wasserstoff, niederes Alkyl, Aryl oder Aryl-niederes-alkyl bedeutet;

$R^{12}$ niederes Alkyl oder niederes Alkoxy-niederes-alkyl bedeutet;

$R^{13}$ niederes Alkyl bedeutet;
$R^{14}$ und $R^{15}$ je Wasserstoff, niederes Alkyl, Aryl oder Aryl-niederes-alkyl bedeuten: und
$R^{16}$ Wasserstoff, niederes Alkyl, niederes Alkoxy-niederes-alkyl, niederes Alkenyl, niederes Alkinyl, Acyl oder Aryl-niederes-alkyl bedeuten;
sowie pharmazeutisch verwendbare Salze von sauren Verbindungen der Formel I mit Basen und von basischen Verbindungen der Formel I mit Säuren; mit Ausnahme von
- 4-Methoxy-1-(3-methylphenyl)-2-butin-1-on;
- 4-Methoxy-1-(4-methoxyphenyl)-4-methyl-2-pentin-1-on;
- 4-Methoxy-4-methyl-1-(3-methylphenyl)-2-pentin-1-on;
- 3-(2,6-Dimethoxybenzoyl)propiolsäuremethylester und
- N,N-Diisopropyl-3-(2-hydroxy-5-methylbenzoyl)propiolamid.

2. Verbindungen gemäss Anspruch 1, worin $R^1$, $R^2$, $R^3$, $R^4$ und $R^5$ je Wasserstoff, niederes Alkyl, Hydroxy, niederes Alkoxy, niederes Alkylthio oder di-niederes-Alkylamino oder zwei benachbarte dieser Substituenten zusammen niederes Alkylen oder niederes Alkylendioxy bedeuten, wobei von den Substituenten $R^1$ bis $R^5$ mindestens einer von Wasserstoff verschieden ist und mindestens zwei Wasserstoff bedeuten.

3. Verbindungen gemäss Anspruch 2, worin $R^1$ Wasserstoff bedeutet.

4. Verbindungen gemäss Anspruch 3, worin $R^1$ und $R^2$ je Wasserstoff und $R^3$, $R^4$ und $R^5$ je niederes Alkoxy oder $R^1$ und $R^5$ je Wasserstoff und $R^2$, $R^3$ und $R^4$ je niederes Alkoxy oder $R^1$, $R^3$ und $R^4$ je Wasserstoff und $R^2$ und $R^5$ je niederes Alkoxy oder $R^1$, $R^2$ und $R^5$ je Wasserstoff und $R^3$ und $R^4$ zusammen niederes Alkylendioxy oder niederes Alkylen oder $R^1$, $R^2$, $R^4$ und $R^5$ je Wasserstoff und $R^3$ Halogen, niederes Alkyl, Hydroxy, niederes Alkoxy, niederes Alkylthio oder di-niederes Alkylamino bedeuten und worin $R^6$ einen Rest der Formel (a), (b), (c), (d), (e) oder (f) bedeutet, wobei $R^7$ Wasserstoff, niederes Alkyl, niederes Alkoxy-niederes-alkoxy-niederes-alkyl, Aryl-niederes-alkyl oder niederes Alkenyl, $R^8$ und $R^9$ je niederes Alkyl, $R^{10}$ Wasserstoff, $R^{11}$ Wasserstoff, $R^{12}$ niederes Alkyl, $R^{13}$ niederes Alkyl, $R^{14}$ Wasserstoff, $R^{15}$ Wasserstoff oder niederes Alkyl und $R^{16}$ Wasserstoff, niederes Alkenyl, niederes Alkoxy-niederes-alkyl oder Acyl bedeuten.

5. Verbindungen gemäss einem der Ansprüche 1 bis 4, worin $R^6$ einen Rest der Formel (a), (b), (c), (d) oder (f) bedeutet, wobei $R^7$ Wasserstoff, niederes Alkyl, niederes Alkoxy-niederes-alkoxy-niederes-alkyl, Aryl-niederes-alkyl oder niederes Alkenyl, $R^8$ und $R^9$ je niederes Alkyl, $R^{10}$ Wasserstoff, $R^{11}$ Wasserstoff, $R^{12}$ niederes Alkyl, $R^{14}$ Wasserstoff, $R^{15}$ Wasserstoff oder niederes Alkyl und $R^{16}$ Wasserstoff, niederes Alkoxy-niederes-alkyl oder Acyl bedeuten.

6. Verbindungen gemäss einem der Ansprüche 1 bis 5, worin $R^1$ und $R^2$ je Wasserstoff und $R^3$, $R^4$ und $R^5$ je Methoxy oder $R^1$ und $R^5$ je Wasserstoff und $R^2$, $R^3$ und $R^4$ je Methoxy oder $R^1$, $R^3$ und $R^4$ je Wasserstoff und $R^2$ und $R^5$ je Methoxy oder $R^1$, $R^2$ und $R^5$ je Wasserstoff und $R^3$ und $R^4$ zusammen Methylendioxy oder $R^1$, $R^2$, $R^4$ und $R^5$ je Wasserstoff und $R^3$ Chlor, Fluor, Methyl, Hydroxy, Methoxy oder Methylthio bedeuten und worin $R^6$ einen Rest der Formel (a), (b) oder (f) bedeutet, wobei $R^7$ Wasserstoff, Methyl oder Methoxyäthoxyäthyl, $R^8$ und $R^9$ je Methyl, $R^{14}$ Wasserstoff, $R^{15}$ Wasserstoff oder Methyl und $R^{16}$ Wasserstoff oder 1-Aethoxyäthyl bedeuten.

7. 3-[3,4-(Methylendioxy)benzoyl]propiolsäure.

8. 4-Hydroxy-1-(3,4,5-trimethoxyphenyl)-2-butin-1-on.

9. 3-(4-Methoxybenzoyl)propiolsäure.

10. 3-(2,3,4-Trimethoxybenzoyl)propiolsäuremethylester.

11. 3-(4-Hydroxybenzoyl)propiolsäuremethylester.

12. 3-(3,4,5-Trimethoxybenzoyl)propiolsäure-[2-(2-methoxyäthoxy)äthyl]ester.

13. 3-(4-Methoxybenzoyl)propiolsäure-[2-(2-methoxyäthoxy)äthyl]ester.

14. 3-(2,5-Dimethoxybenzoyl)propiolsäuremethylester.

15. 3-(2,5-Dimethoxybenzoyl)propiolsäure.

16. 3-[3,4-(Methylendioxy)benzoyl]propiolsäure-[2-(2-methoxyäthoxy)äthyl]ester.

17. 3-(2,3,4-Trimethoxybenzoyl)propiolsäure;
3-(3,4,5-Trimethoxybenzoyl)propiolsäure;
3-(4-Fluorbenzoyl)propiolsäuremethylester;
3-(3,4,5-Trimethoxybenzoyl)propiolsäuremethylester;
N,N-Dimethyl-3-(3,4,5-trimethoxybenzoyl)propiolamid;
1-(4-Fluorphenyl)-4-hydroxy-2-butin-1-on;
4-Hydroxy-1-(4-methylphenyl)-2-butin-1-on;
4-(1-Aethoxyäthoxy)-1-(4-fluorphenyl)-2-butin-1-on;
1-(4-Chlorphenyl)-4-hydroxy-2-butin-1-on;

4-Hydroxy-1-(4-methoxyphenyl)-2-butin-1-on;
4-Hydroxy-1-[3,4-(methylendioxy)phenyl]-2-butin-1-on;
4-Hydroxy-1-(3,4,5-trimethoxyphenyl)-2-pentin-1-on;
4-(1-Aethoxyäthoxy)-1-(2,3,4-trimethoxyphenyl)-2-butin-1-on;
4-Hydroxy-1-(2,3,4-trimethoxyphenyl)-2-butin-1-on; und
4-Hydroxy-1-[4-(methylthio)phenyl]-2-butin-1-on.

18. Verbindungen der allgemeinen Formel

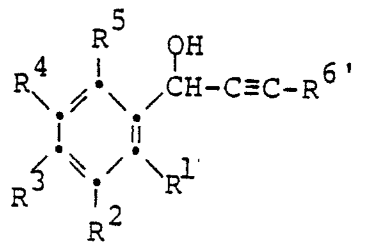

II

worin $R^1$, $R^2$, $R^3$, $R^4$ und $R^5$ die in Anspruch 1 angegebene Bedeutung besitzen und $R^{6'}$ Wasserstoff, niederes Alkyl, einen Rest der in Anspruch 1 definierten Formel (a), (b), (c), (d) oder (e) oder einen Rest der Formel

$-C(R^{14})(R^{15})OR^{16'}$ (f')

bedeutet, worin $R^{14}$ und $R^{15}$ die in Anspruch 1 angegebene Bedeutung besitzen und $R^{16'}$ die in Anspruch 1 für $R^{16}$ definierte Bedeutung besitzt, jedoch nicht Wasserstoff bedeutet wenn $R^{14}$ und/oder $R^{15}$ Wasserstoff bedeutet mit Ausnahme von

- 4-(3,5-Dimethoxyphenyl)-4-hydroxy-2-butinsäureäthylester;
- 4-(2,5-Dimethoxyphenyl)-4-hydroxy-2-butinsäureäthylester;
- 4-(2-Methoxyphenyl)-4-hydroxy-2-butinsäureäthylester;
- 4-(2-Benzyloxy-6-methoxyphenyl)-4-hydroxy-2-butinsäuremethylester;
- 4-(2-Benzyloxy-6-methoxyphenyl)-4-hydroxy-2-butinsäure; und
- 4-(2,6-Dimethoxyphenyl)-4-hydroxy-2-butinsäure.

19. Verbindungen gemäss Anspruch 18, worin $R^1$, $R^2$, $R^3$, $R^4$ und $R^5$ je Wasserstoff, niederes Alkyl, Hydroxy, niederes Alkoxy, niederes Alkylthio oder di-niederes-Alkylamino oder zwei benachbarte dieser Substituenten zusammen niederes Alkylen oder niederes Alkylendioxy bedeuten, wobei von den Substituenten $R^1$ bis $R^5$ mindestens einer von Wasserstoff verschieden ist und mindestens zwei Wasserstoff bedeuten.

20. Verbindungen gemäss Anspruch 19, worin $R^1$ Wasserstoff bedeutet.

21. Verbindungen gemäss Anspruch 20, worin $R^1$ und $R^2$ je Wasserstoff und $R^3$, $R^4$ und $R^5$ je niederes Alkoxy oder $R^1$ und $R^5$ je Wasserstoff und $R_2$, $R^3$ und $R^4$ je niederes Alkoxy oder $R^1$, $R_2$ und $R^5$ je Wasserstoff und $R^3$ und $R^4$ zusammen niederes Alkylendioxy oder niederes Alkylen oder $R^1$, $R^2$, $R^4$ und $R^5$ je Wasserstoff und $R^3$ Halogen, niederes Alkyl, Hydroxy, niederes Alkoxy, niederes Alkylthio oder di-niederes Alkylamino bedeuten

22. Verbindungen gemäss einem der Ansprüche 18 bis 21, worin $R^{6'}$ einen Rest der in Anspruch 1 definierten Formel (a) bedeutet.

23. Verbindungen gemäss Anspruch 22, worin $R^7$ niederes Alkyl oder niederes Alkoxy-niederes-alkoxy-niederes-alkyl bedeutet.

24. 4-Hydroxy-4-[3,4-methylendioxy)phenyl]-2-butinsäuremethylester.

25. 4-Hydroxy-4-(3,4,5-trimethoxyphenyl)-2-butinsäure-[2-(2-methoxyäthoxy)äthyl]ester.

26. Verbindungem gemäss einem der Ansprüche 1 bis 6 und 8 bis 25 sowie

- 4-Methoxy-1-(3-methylphenyl)-2-butin-1-on,
- 4-Methoxy-1-(4-methoxyphenyl)-4-methyl-2-pentin-1-on,
- 4-Methoxy-4-methyl-1-(3-methylphenyl)-2-pentin-1-on,
- 3-(2,6-Dimethoxybenzoyl)propiolsäuremethylester,
- N,N-Diisopropyl-3-(2-hydroxy-5-methylbenzoyl)propiolamid,
- 4-(3,5-Dimethoxyphenyl)-4-hydroxy-2-butinsäureäthylester,
- 4-(2,5-Dimethoxyphenyl)-4-hydroxy-2-butinsäureäthylester,
- 4-(2-Methoxyphenyl)-4-hydroxy-2-butinsäureäthylester,

- 4-(2-Benzyloxy-6-methoxyphenyl)-4-hydroxy-2-butinsäuremethylester.
- 4-(2-Benzyloxy-6-methoxyphenyl)-4-hydroxy-2-butinsäure; und
- 4-(2,6-Dimethoxyphenyl)-4-hydroxy-2-butinsäure zur Anwendung als therapeutische Wirkstoffe, insbesondere als mucosaprotektive und/oder magensäuresekretionshemmende Wirkstoffe.

27. 3-[3,4-(Methylendioxy)benzoyl]propiolsäure zur Anwendung als therapeutischer Wirkstoff, insbesondere als mucosaprotektiver und/oder magensäuresekretionshemmender Wirkstoff.

28. Verfahren zur Herstellung von Verbindungen gemäss einem der Ansprüche 1 bis 17, dadurch gekennzeichnet, dass man

a) eine Verbindung der allgemeinen Formel

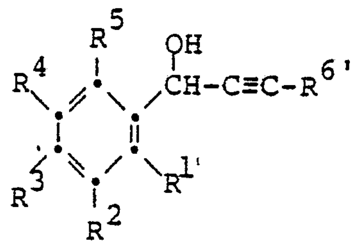

II

worin $R^1$, $R^2$, $R^3$, $R^4$ und $R^5$ die in Anspruch 1 angegebene Bedeutung besitzen und $R^{6'}$ Wasserstoff, niederes Alkyl, einen Rest der in Anspruch 1 definierten Formel (a), (b), (c), (d) oder (e) oder einen Rest der Formel

$-C(R^{14})(R^{15})OR^{16'}$ (f')

bedeutet, worin $R^{14}$ und $R^{15}$ die in Anspruch 1 angegebene Bedeutung besitzen und $R^{16'}$ die in Anspruch 1 für $R^{16}$ definierte Bedeutung besitzt, jedoch nicht Wasserstoff bedeutet wenn $R^{14}$ und/oder $R^{15}$ Wasserstoff bedeutet,
oxydiert; oder

b) von einer Verbindung der allgemeinen Formel

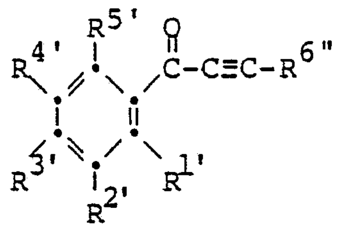

III

worin $R^{1'}$, $R^{2'}$, $R^{3'}$, $R^{4'}$ und $R^{5'}$ die in Anspruch 1 für $R^1$, $R^2$, $R^3$, $R^4$ und $R^5$ angegebene Bedeutung besitzen und maximal drei davon zusätzlich geschütztes Hydroxy, geschütztes Amino oder geschütztes niederes Alkylamino bedeuten können, $R^{6''}$ einen Rest der in Anspruch 1 definierten Formel (a), (b), (c), (d) oder (e) oder einen Rest der Formel

$-C(R^{14})(R^{15})OR^{16''}$ (f'')

bedeutet, worin $R^{14}$ und $R^{15}$ die in Anspruch 1 angegebene Bedeutung besitzen und $R^{16''}$ die in Anspruch 1 für $R^{16}$ angegebene Bedeutung besitzt und zusätzlich eine Schutzgruppe bedeuten kann, wobei das Molekül mindestens eine Schutzgruppe enthält,
die Schutzgruppe(n) abspaltet; oder

c) Eine Verbindung der allgemeinen Formel

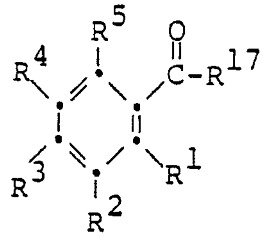

IV

worin $R^1$, $R^2$, $R^3$, $R^4$ und $R^5$ die in Anspruch 1 angegebene Bedeutung besitzen und $R^{17}$ eine Abgangsgruppe bedeutet,
mit einer Verbindung der allgemeinen Formel

$HC{\equiv}C\text{-}R^{6'''}$ V

worin $R^{6'''}$ einen Rest der in Anspruch 1 definierten Formel (a), (b), (d), (e) oder (f) bedeutet,
umsetzt; oder

d) eine Verbindung der Formel I, worin $R^6$ einen Rest der in Anspruch 1 definierten Formel (a) bedeutet, wobei $R^7$ von Wasserstoff verschieden ist, zur entsprechenden Carbonsäure spaltet; oder

e) eine Verbindung der Formel I, worin $R^6$ einen Rest der in Anspruch 1 definierten Formel (f) bedeutet, wobei $R^{15}$ Wasserstoff bedeutet, acyliert; oder

f) eine Verbindung der Formel I, worin $R^6$ einen Rest der in Anspruch 1 definierten Formel (d) bedeutet, in die entsprechende Verbindung der Formel I, worin $R^6$ einen Rest der Formel (c) bedeutet, überführt; oder

g) eine saure Verbindung der Formel I mit einer Base bzw. eine basische Verbindung der Formel I mit einer Säure in ein pharmazeutisch verwendbares Salz überführt.

29. Verfahren gemäss Anspruch 28, dadurch gekennzeichnet, dass man 3-[3,4-(Methylendioxy)-benzoyl]propionsäure herstellt.

30. Verfahren zur Herstellung von Verbindungen gemäss einem der Ansprüche 18 bis 25, dadurch gekennzeichnet, dass man

aa) eine Verbindung der allgemeinen Formel

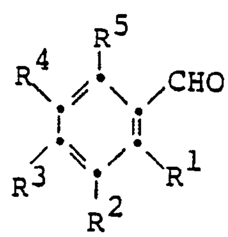

VI

worin $R^1$, $R^2$, $R^3$, $R^4$ und $R^5$ die in Anspruch 1 angegebene Bedeutung besitzen,
mit einer Verbindung der Formel

$HC{\equiv}C\text{-}R^{6iv}$ Va

worin $R^{6iv}$ einen Rest der in Anspruch 1 definierten Formel (a), (b), (d) oder (e) oder der in Anspruch 28 definierten Formel (f') bedeutet,
umsetzt; oder

bb) von einer Verbindung der allgemeinen Formel

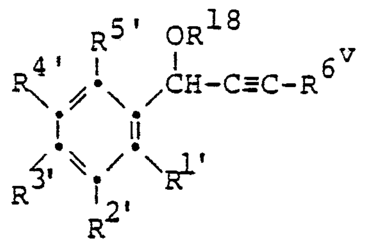

VII

worin $R^{1'}$, $R^{2'}$, $R^{3'}$, $R^{4'}$ und $R^{5'}$ die in Anspruch 18 angegebene Bedeutung besitzen, $R^{6v}$ einen Rest der in Anspruch 1 definierten Formel (a), (b) oder (c) der einen Rest der Formel

$-C(R^{14})(R^{15})OR^{16'''}$ (f''')

bedeutet, wobei $R^7$ und $R^{10}$ von Wasserstoff verschieden sind, $R^{14}$ und $R^{15}$ die in Anspruch 18 und $R^{16'''}$ die in Anspruch 18 für $R^{16'}$ angegebene Bedeutung besitzen, wobei aber $R^{16'''}$ zusätzlich noch eine Schutzgruppe bedeuten kann, und $R^{18}$ eine Schutzgruppe bedeutet,
die Schutzgruppe(n) abspaltet.

31. Verfahren gemäss Anspruch 30, dadurch gekennzeichnet, dass man nach Variante aa) arbeitet, jedoch an Stelle einer Verbindung der Formel Va ein reaktionsfähiges Derivat davon einsetzt.

32. Verfahren gemäss Anspruch 31, dadurch gekennzeichnet, dass man ein Trimethylsilylderivat einer Verbindung der Formel Va einsetzt.

33. Arzneimittel, enthaltend eine Verbindung gemäss einem der Ansprüche 1 bis 6 und 8 bis 25 oder
- 4-Methoxy-1-(3-methylphenyl)-2-butin-1-on oder
- 4-Methoxy-1-(4-methoxyphenyl)-4-methyl-2-pentin-1-on oder
- 4-Methoxy-4-methyl-1-(3-methylphenyl)-2-pentin-1-on oder
- 3-(2,6-Dimethoxybenzoyl)propiolsäuremethylester oder
- N,N-Diisopropyl-3-(2-hydroxy-5-methylbenzoyl)propiolamid oder
- 4-(3,5-Dimethoxyphenyl)-4-hydroxy-2-butinsäureäthylester oder
- 4-(2,5-Dimethoxyphenyl)-4-hydroxy-2-butinsäureäthylester oder
- 4-(2-Methoxyphenyl)-4-hydroxy-2-butinsäureäthylester oder
- 4-(2-Benzyloxy-6-methoxyphenyl)-4-hydroxy-2-butinsäuremethylester oder
- 4-(2-Benzyloxy-6-methoxyphenyl)-4-hydroxy-2-butinsäure oder
- 4-(2,6-Dimethoxyphenyl)-4-hydroxy-2-butinsäure und ein therapeutisch inertes Excipiens.

34. Arzneimittel, enthaltend 3-[3,4-(Methylendioxy)benzoyl]propiolsäure und ein therapeutisch inertes Excipiens.

35. Arzneimittel gemäss Anspruch 33 oder 34 mit mucosaprotektiven und/oder magensäuresekretionshemmenden Eigenschaften zur Bekämpfung oder Verhütung von Ulcus ventriculi und/oder duodeni.

36. Verwendung von Verbindungen gemäss einem der Ansprüche 1 bis 6 und 8 bis 25 sowie von
- 4-Methoxy-1-(3-methylphenyl)-2-butin-1-on,
- 4-Methoxy-1-(4-methoxyphenyl)-4-methyl-2-pentin-1-on,
- 4-Methoxy-4-methyl-1-(3-methylphenyl)-2-pentin-1-on,
- 3-(2,6-Dimethoxybenzoyl)propiolsäuremethylester,
- N,N-Diisopropyl-3-(2-hydroxy-5-methylbenzoyl)propiolamid,
- 4-(3,5-Dimethoxyphenyl)-4-hydroxy-2-butinsäureäthylester,
- 4-(2,5-Dimethoxyphenyl)-4-hydroxy-2-butinsäureäthylester,
- 4-(2-Methoxyphenyl)-4-hydroxy-2-butinsäureäthylester,
- 4-(2-Benzyloxy-6-methoxyphenyl)-4-hydroxy-2-butinsäuremethylester,
- 4-(2-Benzyloxy-6-methoxyphenyl)-4-hydroxy-2-butinsäure; und
- 4-(2,6-Dimethoxyphenyl)-4-hydroxy-2-butinsäure bei der Bekämpfung oder Verhütung von Krankheiten, insbesondere von Ulcus ventriculi und/oder duodeni, bzw. zur Herstellung von Arzneimitteln gegen Ulcus ventriculi und/oder duodeni.

37. Verwendung von 3-[3,4-(Methylendioxy)benzoyl]propiolsäure bei der Bekämpfung oder Verhütung von Krankheiten, insbesondere von Ulcus ventriculi und/oder duodeni, bzw. zur Herstellung von Arzneimitteln gegen Ulcus ventriculi und/oder duodeni.

Patentansprüche für die folgenden Vertragsstaaten: ES, GR.

1. Verfahren zur Herstellung von Propiolophenonderivaten der allgemeinen Formel

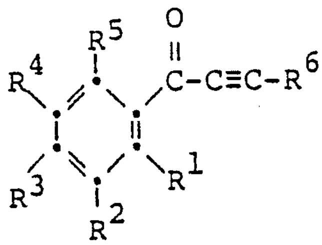

I

worin
$R^1$, $R^2$, $R^3$, $R^4$ und $R^5$ je Wasserstoff, Halogen, niederes Alkyl, niederes Alkoxy-niederes-alkyl, Hydroxy, niederes Alkoxy, niederes Alkenyloxy, niederes Alkinyloxy, niederes Alkoxy-niederes-alkoxy, Acyloxy, Aryl-niederes-alkoxy, niederes Alkylthio, niederes Alkoxy-niederes-alkylthio, niederes Alkenylthio, niederes Alkinylthio, Aryl-niederes-alkylthio, gegebenenfalls substituiertes Amino oder Trifluormethyl oder zwei benachbarte dieser Substituenten gemeinsam und zusammen mit den Kohlenstoffatomen, an welche sie gebunden sind, einen 5-bis 7-gliedrigen Ring bedeuten, wobei von den Substituenten $R^1$ bis $R^5$ mindestens zwei Wasserstoff bedeuten und mindestens einer von Wasserstoff verschieden ist; und $R^6$ Wasserstoff, niederes Alkyl oder einen Rest der Formel

$-COOR^7$, (a) $-CONR^8R^9$, (b) $-C(R^{10})=O$, (c)

$-C(R^{11})(OR^{12})_2$, (d) $-C(OR^{13})_3$ (e) oder

$-C(R^{14})(R^{15})OR^{16}$ (f)

bedeutet;
$R^7$ Wasserstoff, niederes Alkyl, niederes Alkenyl, niederes Alkoxy-niederes-alkyl, niederes Alkoxy-niederes-alkoxy-niederes-alkyl, Aryl oder Aryl-niederes-alkyl bedeutet;
$R^8$ und $R^9$ je Wasserstoff oder niederes Alkyl oder gemeinsam und zusammen mit dem Stickstoffatom einen 5-bis 7-gliedrigen gesättigten heterocyclischen Rest bedeuten;
$R^{10}$ Wasserstoff, niederes Alkyl, Aryl oder Aryl-niederes-alkyl bedeutet;
$R^{11}$ Wasserstoff, niederes Alkyl, Aryl oder Aryl-niederes-alkyl bedeutet;
$R^{12}$ niederes Alkyl oder niederes Alkoxy-niederes-alkyl bedeutet;
$R^{13}$ niederes Alkyl bedeutet;
$R^{14}$ und $R^{15}$ je Wasserstoff, niederes Alkyl, Aryl oder Aryl-niederes-alkyl bedeuten; und
$R^{16}$ Wasserstoff, niederes Alkyl, niederes Alkoxy-niederes-alkyl, niederes Alkenyl, niederes Alkinyl, Acyl oder Aryl-niederes-alkyl bedeuten;
sowie von pharmazeutisch verwendbaren Salzen von sauren Verbindungen der Formel I mit Basen und von basischen Verbindungen der Formel I mit Säuren; mit Ausnahme von
- 4-Methoxy-1-(3-methylphenyl)-2-butin-1-on;
- 4-Methoxy-1-(4-methoxyphenyl)-4-methyl-2-pentin-1-on;

- 4-Methoxy-4-methyl-1-(3-methylphenyl)-2-pentin-1-on;
- 3-(2,6-Dimethoxybenzoyl)propiolsäuremethylester und
- N,N-Diisopropyl-3-(2-hydroxy-5-methylbenzoyl)propiolamid, dadurch gekennzeichnet, dass man

a) eine Verbindung der allgemeinen Formel

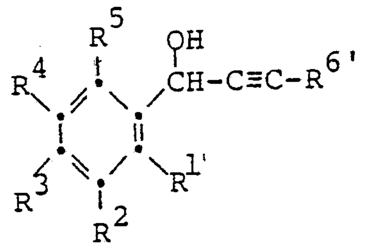

II

worin $R^1$, $R^2$, $R^3$, $R^4$ und $R^5$ obige Bedeutung besitzen und $R^{6'}$ Wasserstoff, niederes Alkyl, einen Rest der oben definierten Formel (a), (b), (c), (d) oder (e) oder einen Rest der Formel

$-C(R^{14})(R^{15})OR^{16'}$ (f')

bedeutet, worin $R^{14}$ und $R^{15}$ obige Bedeutung besitzen und $R^{16'}$ die oben für $R^{16}$ definierte Bedeutung besitzt, jedoch nicht Wasserstoff bedeutet wenn $R^{14}$ und/oder $R^{15}$ Wasserstoff bedeutet,
oxydiert; oder

b) von einer Verbindung der allgemeinen Formel

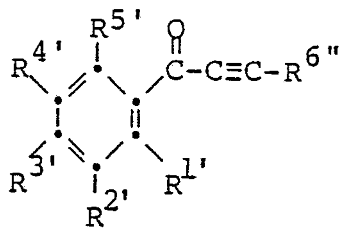

III

worin $R^{1'}$, $R^{2'}$, $R^{3'}$, $R^{4'}$ und $R^{5'}$ die oben für $R^1$, $R^2$, $R^3$, $R^4$ und $R^5$ angegebene Bedeutung besitzen und maximal drei davon zusätzlich geschütztes Hydroxy, geschütztes Amino oder geschütztes niederes Alkylamino bedeuten können, $R^{6''}$ einen Rest der oben definierten Formel (a), (b), (c), (d) oder (e) oder einen Rest der Formel

$-C(R^{14})(R^{15})OR^{16''}$ (f'')

bedeutet, worin $R^{14}$ und $R^{15}$ obige Bedeutung besitzen und $R^{16''}$ die oben für $R^{16}$ angegebene Bedeutung besitzt und zusätzlich eine Schutzgruppe bedeuten kann, wobei das Molekül mindestens eine Schutzgruppe enthält, die Schutzgruppe(n) abspaltet; oder

c) eine Verbindung der allgemeinen Formel

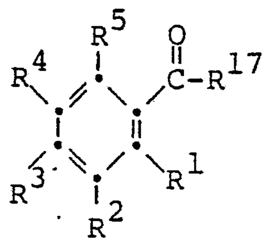

IV

worin $R^1$, $R^2$, $R^3$, $R^4$ und $R^5$ obige Bedeutung besitzen und $R^{17}$ eine Abgangsgruppe bedeutet,
mit einer Verbindung der allgemeinen Formel

$HC{\equiv}C\text{-}R^{6'''}$ V

worin $R^{6'''}$ einen Rest der oben definierten Formel (a), (b), (d), (e) oder (f) bedeutet, umsetzt; oder

d) eine Verbindung der Formel I, worin $R^6$ einen Rest der oben definierten Formel (a) bedeutet, wobei $R^7$ von Wasserstoff verschieden ist, zur entsprechenden Carbonsäure spaltet; oder

e) eine Verbindung der Formel I, worin $R^6$ einen Rest der oben definierten Formel (f) bedeutet, wobei $R^{16}$ Wasserstoff bedeutet, acyliert; oder

f) eine Verbindung der Formel I, worin $R^6$ einen Rest der oben definierten Formel (d) bedeutet, in die entsprechende Verbindung der Formel I, worin $R^6$ einen Rest der Formel (c) bedeutet, überführt; oder

g) eine saure Verbindung der Formel I mit einer Base bzw. eine basische Verbindung der Formel I mit einer Säure in ein pharmazeutisch verwendbares Salz überführt.

2. Verfahren gemäss Anspruch 1, dadurch gekennzeichnet, dass man eine Verbindung der Formel I herstellt, worin $R^1$, $R^2$, $R^3$, $R^4$ und $R^5$ je Wasserstoff, niederes Alkyl, Hydroxy, niederes Alkoxy, niederes Alkylthio oder di-niederes-Alkylamino oder zwei benachbarte dieser Substituenten zusammen niederes Alkylen oder niederes Alkylendioxy bedeuten, wobei von den Substituenten $R^1$ bis $R^5$ mindestens einer von Wasserstoff verschieden ist und mindestens zwei Wasserstoff bedeuten.

3. Verfahren gemäss Anspruch 2, dadurch gekennzeichnet, dass man eine Verbindung der Formel I herstellt, worin $R^1$ Wasserstoff bedeutet.

4. Verfahren gemäss Anspruch 3, dadurch gekennzeichnet, dass man eine Verbindung der Formel I herstellt, worin $R^1$ und $R^2$ je Wasserstoff und $R^3$, $R^4$ und $R^5$ je niederes Alkoxy oder $R^1$ und $R^5$ je Wasserstoff und $R^2$, $R^3$ und $R^4$ je niederes Alkoxy oder $R^1$, $R^3$ und $R^4$ je Wasserstoff und $R^2$ und $R^5$ je niederes Alkoxy oder $R^1$, $R^2$ und $R^5$ je Wasserstoff und $R^3$ und $R^4$ zusammen niederes Alkylendioxy oder niederes Alkylen oder $R^1$, $R^2$, $R^4$ und $R^5$ je Wasserstoff und $R^3$ Halogen, niederes Alkyl, Hydroxy, niederes Alkoxy, niederes Alkylthio oder di-niederes Alkylamino bedeuten und worin $R^6$ einen Rest der Formel (a), (b), (c), (d), (e) oder (f) bedeutet, wobei $R^7$ Wasserstoff, niederes Alkyl, niederes Alkoxy-niederes-alkoxy-niederes-alkyl, Aryl-niederes-alkyl oder niederes Alkenyl, $R^8$ und $R^9$ je niederes Alkyl, $R^{10}$ Wasserstoff, $R^{11}$ Wasserstoff, $R^{12}$ niederes Alkyl, $R^{13}$ niederes Alkyl, $R^{14}$ Wasserstoff, $R^{15}$ Wasserstoff oder niederes Alkyl und $R^{16}$ Wasserstoff, niederes Alkenyl, niederes Alkoxy-niederes-alkyl oder Acyl bedeuten.

5. Verfahren gemäss einem der Ansprüche 1 bis 4, dadurch gekennzeichnet, dass man eine Verbindung der Formel I herstellt, worin $R^6$ einen Rest der Formel (a), (b), (c), (d) oder (f) bedeutet, wobei $R^7$ Wasserstoff, niederes Alkyl, niederes Alkoxy-niederes-alkoxy-niederes-alkyl, Aryl-niederes-alkyl oder niederes Alkenyl, $R^8$ und $R^9$ je niederes Alkyl, $R^{10}$ Wasserstoff, $R^{11}$ Wasserstoff, $R^{12}$ niederes Alkyl, $R^{14}$ Wasserstoff, $R^{15}$ Wasserstoff oder niederes Alkyl und $R^{16}$ Wasserstoff, niederes Alkoxy-niederes-alkyl oder Acyl bedeuten.

6. Verfahren gemäss einem der Ansprüche 1 bis 5, dadurch gekennzeichnet, dass man eine Verbindung der Formel I herstellt, worin $R^1$ und $R^2$ je Wasserstoff und $R^3$, $R^4$ und $R^5$ je Methoxy oder $R^1$ und $R^5$ je Wasserstoff und $R^2$, $R^3$ und $R^4$ je Methoxy oder $R^1$, $R^3$ und $R^4$ je Wasserstoff und $R^2$ und $R^5$ je Methoxy oder $R^1$, $R^2$ und $R^5$ je Wasserstoff und $R^3$ und $R^4$ zusammen Methylendioxy oder $R^1$, $R^2$, $R^4$ und $R^5$ je Wasserstoff und $R^3$ Chlor, Fluor, Methyl, Hydroxy, Methoxy oder Methylthio bedeuten und worin $R^6$ einen Rest der Formel (a), (b) oder (f) bedeutet, wobei $R^7$ Wasserstoff, Methyl oder Methoxyäthoxyäthyl, $R^8$ und $R^9$ je Methyl, $R^{14}$ Wasserstoff, $R^{15}$ Wasserstoff oder Methyl und $R^{16}$ Wasserstoff oder 1-Aethoxyäthyl bedeuten.

7. Verfahren gemäss Anspruch 1, dadurch gekennzeichnet, dass man 3-[3,4-(Methylendioxy)benzoyl]-propiolsäure herstellt.

8. Verfahren gemäss Anspruch 1, dadurch gekennzeichnet, dass man 4-Hydroxy-1-(3,4,5-trimethoxyphenyl)-2-butin-1-on herstellt.

9. Verfahren gemäss Anspruch 1, dadurch gekennzeichnet, dass man 3-(4-Methoxybenzoyl)-propiolsäure herstellt.

10. Verfahren gemäss Anspruch 1, dadurch gekennzeichnet dass man 3-(2,3,4-Trimethoxybenzoyl)-propiolsäuremethylester herstellt.

11. Verfahren gemäss Anspruch 1, dadurch gekennzeichnet, dass man 3-(4-Hydroxybenzoyl)-propiolsäuremethylester herstellt.

12. Verfahren gemäss Anspruch 1, dadurch gekennzeichnet, dass man 3-(3,4,5-Trimethoxybenzoyl)-propiolsäure-[2-(2-methoxyäthoxy)äthyl]ester herstellt.

13. Verfahren gemäss Anspruch 1, dadurch gekennzeichnet, dass man 3-(4-Methoxybenzoyl)-propiolsäure-[2-(2-methoxyäthoxy)äthyl]ester herstellt.

14. Verfahren gemäss Anspruch 1, dadurch gekennzeichnet, dass man 3-(2,5-Dimethoxybenzoyl)-propiolsäuremethylester herstellt.

15. Verfahren gemäss Anspruch 1, dadurch gekennzeichnet, dass man 3-(2,5-Dimethoxybenzoyl)-propiolsäure herstellt.

16. Verfahren gemäss Anspruch 1, dadurch gekennzeichnet, dass man 3-[3,4-(Methylendioxy)benzoyl]-propiolsäure-[2-(2-methoxyäthoxy)äthyl]ester herstellt.

17. Verfahren zur Herstellung von Verbindungen der allgemeinen Formel

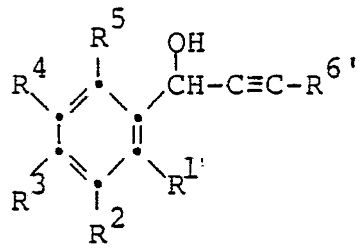

II

worin $R^1$, $R^2$, $R^3$, $R^4$ und $R^5$ die in Anspruch 1 angegebene Bedeutung besitzen und $R^{6'}$ Wasserstoff, niederes Alkyl, einen Rest der in Anspruch 1 definierten Formel (a), (b), (c), (d) oder (e) oder einen Rest der Formel

$-C(R^{14})(R^{15})OR^{16'}$ (f')

bedeutet, worin $R^{14}$ und $R^{15}$ die in Anspruch 1 angegebene Bedeutung besitzen und $R^{16'}$ die in Anspruch 1 für $R^{16}$ definierte Bedeutung besitzt, jedoch nicht Wasserstoff bedeutet wenn $R^{14}$ und/oder $R^{15}$ Wasserstoff bedeutet mit Ausnahme von

- 4-(3,5-Dimethoxyphenyl)-4-hydroxy-2-butinsäureäthylester;
- 4-(2,5-Dimethoxyphenyl)-4-hydroxy-2-butinsäureäthylester;
- 4-(2-Methoxyphenyl)-4-hydroxy-2-butinsäureäthylester;
- 4-(2-Benzyloxy-6-methoxyphenyl)-4-hydroxy-2-butinsäuremethylester;
- 4-(2-Benzyloxy-6-methoxyphenyl)-4-hydroxy-2-butinsäure; und
- 4-(2,6-Dimethoxyphenyl)-4-hydroxy-2-butinsäure, dadurch gekennzeichnet, dass man

aa) eine Verbindung der allgemeinen Formel

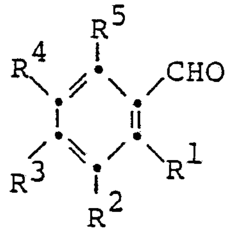

VI

worin $R^1$, $R^2$, $R^3$, $R^4$ und $R^5$ die in Anspruch 1 angegebene Bedeutung besitzen, mit einer Verbindung der Formel

$HC\equiv C-R^{6iv}$ Va

worin $R^{6iv}$ einen Rest der in Anspruch 1 definierten Formel (a), (b), (d) oder (e) oder der oben definierten Formel (f') bedeutet, umsetzt; oder

bb) von einer Verbindung der allgemeinen Formel

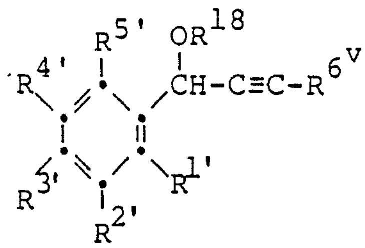

VII

worin $R^{1'}$,$^{2'}$, $R^{3'}$, $R^{4'}$ und $R^{5'}$ die in Anspruch 1 angegebene Bedeutung besitzen, $R^{6v}$ einen Rest der in Anspruch 1 definierten Formel (a), (b) oder (c) oder einen Rest der Formel

$-C(R^{14})(R^{15})OR^{16'''}$ (f''')

bedeutet, wobei $R^{7}$ und $R^{10}$ von Wasserstoff verschieden sind, $R^{14}$ und $R^{15}$ die in Anspruch 1 und $R^{16'''}$ die oben für $R^{16'}$ angegebene Bedeutung besitzen, wobei aber $R^{16'''}$ zusätzlich noch eine Schutzgruppe bedeuten kann, und $R^{18}$ eine Schutzgruppe bedeutet, die Schutzgruppe(n) abspaltet.

18. Verfahren gemäss Anspruch 17, dadurch gekennzeichnet, dass man nach Variante aa) arbeitet jedoch an Stelle einer Verbindung der Formel Va ein reaktionsfähiges Derivat davon einsetzt.

19. Verfahren gemäss Anspruch 18, dadurch gekennzeichnet, dass man ein Trimethylsilylderivat einer Verbindung der Formel Va einsetzt.

20. Verfahren gemäss einem der Ansprüche 17-19, dadurch gekennzeichnet, dass man eine Verbindung der Formel II herstellt, worin $R^1$, $R^2$, $R^3$, $R^4$ und $R^5$ je Wasserstoff, niederes Alkyl, Hydroxy, niederes Alkoxy, niederes Alkylthio oder di-niederes-Alkylamino oder zwei benachbarte dieser Substituenten zusammen niederes Alkylen oder niederes Alkylendioxy bedeuten, wobei von den Substituenten $R^1$ bis $R^5$ mindestens einer von Wasserstoff verschieden ist und mindestens zwei Wasserstoff bedeuten.

21. Verfahren gemäss Anspruch 20, dadurch gekennzeichnet, dass man eine Verbindung der Formel II herstellt, worin $R^1$ Wasserstoff bedeutet.

22. Verfahren gemäss Anspruch 21, dadurch gekennzeichnet, dass man eine Verbindung der Formel II herstellt, worin $R^1$ und $R^2$ je Wasserstoff und $R^3$, $R^4$ und $R^5$ je niederes Alkoxy oder $R^1$ und $R^5$ je Wasserstoff und $R^2$, $R^3$ und $R^4$ je niederes Alkoxy oder $R^1$, $R^2$ und $R^5$ je Wasserstoff und $R^3$ und $R^4$ zusammen niederes Alkylendioxy oder niederes Alkylen oder $R^1$, $R^2$, $R^4$ und $R^5$ je Wasserstoff und $R^3$ Halogen, niederes Alkyl, Hydroxy, niederes Alkoxy, niederes Alkylthio oder di-niederes Alkylamino bedeuten

23. Verfahren gemäss einem der Ansprüche 17 bis 22, dadurch gekennzeichnet, dass man eine Verbindung der Formel II herstellt, worin $R^{6'}$ einen Rest der in Anspruch 1 definierten Formel (a) bedeutet.

24. Verfahren gemäss Anspruch 23, dadurch gekennzeichnet, dass man eine Verbindung der Formel II herstellt, worin $R^7$ niederes Alkyl oder niederes Alkoxy-niederes-alkoxy-niederes-alkyl bedeutet.

25. Verfahren gemäss einem der Ansprüche 17 bis 19, dadurch gekennzeichnet, dass man 4-Hydroxy-4-[3,4-methylendioxy)phenyl]-2-butinsäure-methylester herstellt.

26. Verfahren gemäss einem der Ansprüche 17 bis 19, dadurch gekennzeichnet, dass man 4-Hydroxy-4-(3,4,5-trimethoxyphenyl)-2-butinsäure-[2-(2-methoxyäthoxy)äthyl]ester herstellt.

27. Verfahren zur Herstellung von Arzneimitteln, insbesondere von Arzneimitteln mit mucosaprotektiven und/oder magensäuresekretionshemmenden Eigenschaften, dadurch gekennzeichnet, dass man eine oder mehrere der Verbindungen der in Anspruch 1 bzw. in Anspruch 17 definierten Formel I oder II oder der pharmazeutisch verwendbaren Salze der Verbindungen der Formel I, einschliesslich

- 4-Methoxy-1-(3-methylphenyl)-2-butin-1-on,
- 4-Methoxy-1-(4-methoxyphenyl)-4-methyl-2-pentin-1-on,
- 4-Methoxy-4-methyl-1-(3-methylpenyl)-2-pentin-1-on,
- 3-(2,6-Dimethoxybenzoyl)propiolsäuremethylester,
- N,N-Diisopropyl-3-(2-hydroxy-5-methylbenzoyl)propiolamid,
- 4-(3,5-Dimethoxyphenyl)-4-hydroxy-2-butinsäureäthylester,
- 4-(2,5-Dimethoxyphenyl)-4-hydroxy-2-butinsäureäthylester,
- 4-(2-Methoxyphenyl)-4-hydroxy-2-butinsäureäthylester,
- 4-(2-Benzyloxy-6-methoxyphenyl)-4-hydroxy-2-butinsäuremethylester,
- 4-(2-Benzyloxy-6-methoxyphenyl)-4-hydroxy-2-butinsäure; und

- 4-(2,6-Dimethoxyphenyl)-4-hydroxy-2-butinsäure und erwünschtenfalls einen oder mehrere andere therapeutische Wirkstoffe zusammen mit einem oder mehreren therapeutisch inerten Hilfsstoffen in eine galenische Darreichungsform bringt.

28. Verwendung von Verbindungen der in Anspruch 1 bzw. Anspruch 17 definierten Formeln I und II und von pharmazeutisch verwendbaren Salzen von Verbindungen der Formel I einschliesslich
- 4-Methoxy-1-(3-methylphenyl)-2-butin-1-on,
- 4-Methoxy-1-(4-methoxyphenyl)-4-methyl-2-pentin-1-on,
- 4-Methoxy-4-methyl-1-(3-methylphenyl)-2-pentin-1-on,
- 3-(2,6-Dimethoxybenzoyl)propiolsäuremethylester,
- N,N-Diisopropyl-3-(2-hydroxy-5-methylbenzoyl)propiolamid,
- 4-(3,5-Dimethoxyphenyl)-4-hydroxy-2-butinsäureäthylester,
- 4-(2,5-Dimethoxyphenyl)-4-hydroxy-2-butinsäureäthylester,
- 4-(2-Methoxyphenyl)-4-hydroxy-2-butinsäureäthylester,
- 4-(2-Benzyloxy-6-methoxyphenyl)-4-hydroxy-2-butinsäuremethylester,
- 4-(2-Benzyloxy-6-methoxyphenyl)-4-hydroxy-2-butinsäure; und
- 4-(2,6-Dimethoxyphenyl)-4-hydroxy-2-butinsäure zur Herstellung von Arzneimitteln gegen Ulcus ventriculi und/oder duodeni.